(19)

Europäisches
Patentamt
European
Patent Office
Office européen
des brevets

(11)　**EP 4 523 616 A2**

(12)　　　　　　**EUROPEAN PATENT APPLICATION**

(43) Date of publication:
　　**19.03.2025  Bulletin 2025/12**

(21) Application number: **25154819.4**

(22) Date of filing: **23.11.2016**

(51) International Patent Classification (IPC):
　　***A61B 5/0285*** *(2006.01)*

(52) Cooperative Patent Classification (CPC):
　　**A61B 5/14532; A61B 5/00; A61B 5/02;**
　　**A61B 5/0205; A61B 5/024; A61B 5/0245;**
　　**A61B 5/0285; A61B 5/14546; A61B 5/349;**
　　**A61B 5/7264;** G16H 50/20

(84) Designated Contracting States:
　　**AL AT BE BG CH CY CZ DE DK EE ES FI FR GB**
　　**GR HR HU IE IS IT LI LT LU LV MC MK MT NL NO**
　　**PL PT RO RS SE SI SK SM TR**

(30) Priority:　**23.11.2015  US 201562258956 P**
　　　　　　　**28.09.2016  US 201662401044 P**

(62) Document number(s) of the earlier application(s) in
accordance with Art. 76 EPC:
　　**16869280.4 / 3 379 998**

(71) Applicants:
　　• **Mayo Foundation for Medical Education and**
　　　**Research**
　　　**Rochester, Minnesota 55905 (US)**
　　• **B.G. Negev Technologies and Applications Ltd.,**
　　　**at**
　　　**Ben-Gurion University**
　　　**8410501 Beer Sheva (IL)**

(72) Inventors:
　　• **FRIEDMAN, Paul A.**
　　　**Rochester, 55902-1040 (US)**
　　• **BENNET, Kevin, E.**
　　　**Rochester, 55902-1040 (US)**
　　• **ASIRVATHAM, Samuel, J.**
　　　**Rochester, 55906-8971 (US)**

　　• **BRUCE, Charles, J.**
　　　**Rochester, 55902-4137 (US)**
　　• **ACKERMAN, Michael, J.**
　　　**Rochester, 55902-2913 (US)**
　　• **DILLON, John J.**
　　　**Rochester, 55901 (US)**
　　• **SOMERS, Virend K.**
　　　**Rochester, 55902-1325 (US)**
　　• **SADOT, Dan**
　　　**76965 Kfar Bilu (IL)**
　　• **GEVA, Amir**
　　　**61000 Tel Aviv (IL)**
　　• **SAPIR, Yehu**
　　　**70700 Gedera (IL)**
　　• **ATTIA, Zachi**
　　　**Rochester, 55901 (US)**

(74) Representative: **Birch, Matthew John**
　　**Caldwell IP Ltd**
　　**4th Floor**
　　**36 St James's Street**
　　**London SW1A 1JD (GB)**

Remarks:
　　This application was filed on 29.01.2025 as a
　　divisional application to the application mentioned
　　under INID code 62.

(54)　　**PROCESSING PHYSIOLOGICAL ELECTRICAL DATA FOR ANALYTE ASSESSMENTS**

(57)　　　Among the techniques described herein is a
method that includes obtaining data indicating electro-
cardiogram results from a human. A plurality of beats
represented in the electrocardiogram results can be
identified. For each beat in the plurality of beats represented in the electrocardiogram results, a value for a first
feature of the beat can be determined. Statistical analysis
can be performed on the values for the first feature of the
plurality of beats. An indication of the level of the analyte
within the human can be generated based on a result of
the statistical analysis performed on the values for the
first feature of the plurality of beats. The indication of the
level of the analyte within the human can be provided.

FIG. 1

**EP 4 523 616 A2**

**Description**

## CROSS-REFERENCE TO RELATED APPLICATIONS

This application claims priority to U.S. Application Serial No. 62/258,956, filed on November 23, 2015 and U.S. Application Serial No. 62/401,044, filed on September 28, 2016. The disclosure of the prior applications is considered part of the disclosure of this application, and is incorporated in its entirety into this application.

## TECHNICAL FIELD

This document generally describes computer-based technology for analyzing physiological electrical data (e.g., electrocardiogram data).

## BACKGROUND

Blood potassium levels are tightly homeostatically regulated, and critical for normal physiologic cellular function. See Einhorn LM, Zhan M, Walker LD, Moen MF, Seliger SL, Weir MR, Fink JC: The frequency of hyperkalemia and its significance in chronic kidney disease. Archives of Internal Medicine 169, 1156-1162 (2009); Goyal A, Spertus JA, Gosch K: Serum potassium levels and mortality in acute myocardial infarction. JAMA 307, 157-164, doi:10.1001/jama.2011.1967 (2012). Fluctuations in potassium values are found in many disease states and can expose patients to life threatening arrhythmias. See Gennari, F. J. Hypokalemia. New England Journal of Medicine 339, 451-458, doi:doi:10.1056/NEJM199808133390707 (1998); Weiner, I. D. & Wingo, C. S. Hyperkalemia: a potential silent killer. JOURNAL-AMERICAN SOCIETY OF NEPHROLOGY 9, 1535-1543 (1998); Kovesdy, C. P. Management of hyperkalaemia in chronic kidney disease. Nat Rev Nephrol 10, 653-662, doi:10.1038/nmeph.2014.168 (2014). There is compelling evidence that in patients with renal or cardiac disease, even modest potassium changes may lead to morbidity, hospitalization, and death. See Jain N, Kotla S, Little BB, Weideman RA, Brilakis ES, Reilly RF, Banerjee S: Predictors ofhyperkalemia and death in patients with cardiac and renal disease. The American journal of cardiology 109, 1510-1513 (2012). Moreover, evidence-based therapies used to treat these conditions, including adrenergic blockade, potassium sparing diuretics and renin-angiotensin antagonism, can result in hyper or hypokalemia. After the potassium-sparing diuretic spironolactone was shown to lower heart failure mortality in a randomized prospective trial, hospitalization for hyperkalemia tripled, and mortality doubled. See Juurlink DN, Mamdani MM, Lee DS, Kopp A, Austin PC, Laupacis A, Redelmeier DA: Rates of hyperkalemia after publication of the Randomized Aldactone Evaluation Study. New England Journal of Medicine 351, 543-551 (2004). As the prevalence of these diseases and their risk factors (hypertension and diabetes) rise, and as the population continues to age, increasing numbers of patients will be at risk of hyperkalemia and hypokalemia. See Wild, S., Roglic, G., Green, A., Sicree, R. & King, H. Global prevalence of diabetes estimates for the year 2000 and projections for 2030. Diabetes care 27, 1047-1053 (2004); Roger VL, Go AS, Lloyd-Jones DM, Benjamin EJ, Berry JD, Borden WB, Bravata DM, Dai S, Ford ES, Fox CS, Fullerton HJ, Gillespie C, Hailpem SM, Heit JA, Howard VJ, Kissela BM, Kittner SJ, Lackland DT, Lichtman JH, Lisabeth LD, Makuc DM, Marcus GM, Marelli A, Matchar DB, Moy CS, Mozaffarian D, Mussolino ME, Nichol G, Paynter NP, Soliman EZ, Sorlie PD, Sotoodehnia N, Turan TN, Virani SS, Wong ND, Woo D, Turner MB; American Heart Association Statistics Committee and Stroke Statistics Subcommittee. Heart Disease and Stroke Statistics-2012 Update: A Report From the American Heart Association. Circulation 125, e2-e220, doi:10.1161/CIR.0b013e31823ac046 (2012).

Potassium levels outside the normal range are concerning, as they are usually clinically silent, occurring without warning to the patient or provider in the absence of blood tests. See Gumz, M. L., Rabinowitz, L. & Wingo, C. S. An Integrated View of Potassium Homeostasis. New England Journal of Medicine 373, 60-72, doi:doi:10.1056/NEJMra1313341 (2015).

## SUMMARY

Systems, methods, devices, and other techniques are described herein by which processed ECG signals, or other types of electrogram data, can be used to determine levels of analytes (e.g., potassium) in a patient with clinically meaningful resolution. These techniques can allow for a reliable analyte assessment even when data from just a single ECG lead is collected. In some implementations, a personalized strategy can be employed in which an algorithm is individualized based on seeding blood tests. In some implementations, a global analysis strategy can be employed that requires no blood tests at all. These techniques may thus allow for unobtrusive remote monitored potassium assessment. By enabling both accurate potassium value ascertainment and trend detection, alerts can be issued and interventions initiated, thereby improving clinical outcomes.

Elsewhere, we have described techniques by which, for example, electrical physiological data recorded from a standard 12-lead ECG is analyzed to determine analyte levels in a patient's bloodstream. But the standard 12-lead ECG approach is often diagnostic only after the onset of severe hyper- or hypokalemia. Accordingly, the techniques described herein are aimed at addressing a need for a non-invasive method for measuring potassium prior to clinically significant changes that may lead to arrhythmogenic death, in order to initiate timely lifesaving treatment. See Ingelfinger, J. R. A New Era for the Treatment of Hyperkalemia? New England Journal of Medicine 372, 275-277, doi:doi:10.1056/NEJMe1414112 (2015); *see also* PCT Publication No. WO2015/048514, which is hereby incorporated by reference in its entirety.

In some implementations, these techniques can include noninvasive remote potassium monitoring, which may permit the administration of evidence-based life-saving measures and medications, including recently developed safe and effective potassium lowering medications. *See* Ash, S. R., Singh, B., Lavin, P. T., Stavros, F. & Rasmussen, H. S. A phase 2 study on the treatment of hyperkalemia in patients with chronic kidney disease suggests that the selective potassium trap, ZS-9, is safe and efficient. Kidney Int, doi:10.1038/ki.2014.382 (2015); Packham DK, Rasmussen HS, Lavin PT, El-Shahawy MA, Roger SD, Block G, Qunibi W, Pergola P, Singh B: Sodium Zirconium Cyclosilicate in Hyperkalemia. New England Journal of Medicine 372, 222-231, doi:doi:10.1056/NEJMoa1411487 (2015); Bakris GL, Pitt B, Weir MR, Freeman MW, Mayo MR, Garza D, Stasiv Y, Zawadzki R, Berman L, Bushinsky DA; AMETHYST-DN Investigators. Effect of patiromer on serum potassium level in patients with hyperkalemia and diabetic kidney disease: The amethyst-dn randomized clinical trial. JAMA 314, 151-161, doi:10.1001/jama.2015.7446 (2015); Roscioni, S. S. & Heerspink, H. J. L. Clinical trials: New nonabsorbable potassium-exchange resins in hyperkalaemia Nat Rev Nephrol 11, 205-206, doi:10.1038/nrneph.2014.252 (2015).

Some implementations of the subject matter described herein include a computer-implemented method for assessing a level of an analyte within a human. The method can include obtaining data indicating electrocardiogram results from the human. A plurality of beats represented in the electrocardiogram results can be identified. For each beat in the plurality of beats represented in the electrocardiogram results, a value for a first feature of the beat can be determined. Statistical analysis can be performed on the values for the first feature of the plurality of beats. An indication of the level of the analyte within the human can be generated based on a result of the statistical analysis performed on the values for the first feature of the plurality of beats. The indication of the level of the analyte within the human can be provided. It is noted that although electrocardiogram results are referred to by way of example, other forms of physiological electrical data may be used in some examples (i.e., electrogram data).

These and other implementations can optionally include one or more of the following features.

The analyte can be potassium, magnesium, phosphorous, calcium, bicarbonate, hydrogen ion, or glucose. In some implementations, the analyte is a pharmaceutical (e.g., digoxin, Class I-C anti-arrhythmia drugs, sotalol, dofetilide, amiodarone, beta blockers, calcium channel blockers, tricyclic antidepressants, lithium, and the like).

Determining, for each beat in the plurality of beats represented in the electrocardiogram results, the value for the first feature of the beat can include calculating, for each beat in the plurality of beats, a slope of at least a portion of a T-wave in the beat between the peak of the T-wave and the end of the T-wave.

Determining, for each beat in the plurality of beats represented in the electrocardiogram results, the value for the first feature of the beat can include calculating, for each beat in the plurality of beats, a magnitude of the peak of a T-wave in the beat.

For each beat in the plurality of beats represented in the electrocardiogram results, a value can be determined for a second feature of the beat other than the first feature.

Statistical analysis can be performed on the values for the second feature of the plurality of beats. The indication of the level of the analyte within the human can be generated further based on a result of the statistical analysis performed on the values for the second feature of the plurality of beats. The statistical analysis may be performed with our without signal-averaging techniques. In one example without signal-averaging, values for the first feature may be determined for individual beats represented in the ECG data, rather than for a "composite" beat that has been averaged from multiple different beats and/or from multiple different signals recorded for the same beat. The statistical analysis may then be performed on the values for the first feature of the individual beats. In another example that does utilize signal-averaging techniques, statistical analysis may be performed on values for the first feature of at least some beats that are "composite" representations of multiple individual beats that have been averaged or otherwise combined.

The data indicating the electrocardiogram results from the human can be data recorded from only a single lead of an electrocardiogram device.

The data indicating the electrocardiogram results from the human can be data recorded from a portable electrocardiogram device having less than 12 leads.

The data indicating the electrocardiogram results from the human can be analyzed to identify segments of the electrocardiogram results having a signal quality score that does not meet a threshold signal quality score. Segments of the electrocardiogram results having the signal quality score that does not meet the threshold signal quality score can be discarded.

Identifying the plurality of beats represented in the electrocardiogram results can include identifying beats from segments of the electrocardiogram results other than the discarded segments having the signal quality score that does not meet the threshold signal quality score.

Performing statistical analysis on the values for the first feature of the plurality of beats can include fitting a distribution of the values for the first feature of at least some of the plurality of beats to a probability distribution function.

The probability distribution function can be a normal probability distribution function, a gamma probability distribution function, or a Gaussian probability distribution function.

Generating the indication of the level of the analyte within the human can include comparing the values for the first feature of at least a subset of the plurality of beats to a pre-defined template that maps values for the first feature to levels of the analyte within a human or that maps values derived at least in part from the first feature to levels of the analyte within a human.

The pre-defined template can be generated based on assessments of the level of the analyte within a population of humans other than the human from which the data indicating the electrocardiogram results was obtained.

The pre-defined template can be generated based on assessments of the level of the analyte within the human from which the data indicating the electrocardiogram results was obtained.

Generating the indication of the level of the analyte within the human can include comparing the result of the statistical analysis performed on the values for the first feature of the plurality of beats to a pre-defined template that maps statistical results for historical values for the first feature to levels of the analyte within a human.

The pre-defined template can be generated based on assessments of the level of the analyte within a population of humans other than the human from which the data indicating the electrocardiogram results was obtained.

The pre-defined template can be generated based on assessments of the level of the analyte within the human from which the data indicating the electrocardiogram results was obtained.

The method or operations can be performed at least in part on a mobile computing device of the human. Different divisions of processing among a mobile computing device (or other user/client device) and remote servers are possible. In a first example, the entire algorithm for assessing analyte levels may be performed locally on a mobile device. In a second example, portions of the algorithm may be performed remotely, such as through a cloud-based software-as-a-service (SAAS) platform (e.g., raw ECG data may be transmitted from the mobile device over the Internet to a remote server, and the remote server may process the ECG data to determine an analyte level that is sent back to the mobile device for presentation to a user, or sent to another platform or device for care provider review). In a third example, pre-processing of the ECG data (e.g., artifact detection and rejection) may be performed locally by the mobile device, and the processed ECG data and/or feature values derived from the ECG data may be submitted to a remote server for processing to ultimately determine an analyte level.

Providing the indication of the level of the analyte within the human can include presenting the indication of the level of the analyte on an electronic display of the mobile computing device.

Identifying the plurality of beats can include identifying QRS complexes that occur in at least a subset of the plurality of beats. Identifying the QRS complexes that occur in the at least the subset of the plurality of beats can include identifying features that occur in the electrocardiogram results at a frequency indicated by the human's heart rate or by an estimate of the R-R interval in the electrocardiogram results.

An animation can be generated that shows changes in the electrocardiogram results and changes in the first feature over a period of time.

Determining the values for the first feature of the plurality of beats can include determining initial values for the first feature of the plurality of beats and filtering the initial values.

Filtering the initial values for the first feature can include applying a Kalman filter to the initial values. If the analyte assessment is based on multiple features, the Kalman filter may be applied to values of any of the features individually, or may be applied to any of the features in combination.

The data indicating the electrocardiogram results can be modified by demodulating a QRS envelope of the electrocardiogram results.

Some implementations of the subject matter described herein can include a computer-implemented method. The method can include generating electrocardiogram results from the human; and comparing the results to a signal template to obtain an indication of the level of the analyte in the human, wherein the signal template was generated based on assessments of the level of the analyte within a population of humans other than the human from which the electrocardiogram results were generated.

Some implementations of the subject matter described herein can include one or more non-transitory computer-readable devices having instructions stored thereon that, when executed by one or more processors, cause performance of operations comprising obtaining data indicating electrocardiogram results from the human; identifying a plurality of beats represented in the electrocardiogram results; determining, for each beat in the plurality of beats represented in the electrocardiogram results, a value for a first feature of the beat; performing statistical analysis on the values for the first feature of the plurality of beats; generating an indication of the level of the analyte within the human based on a result of the statistical analysis performed on the values for the first feature of the plurality of beats; and providing the indication of the level of the analyte within the human.

Some implementations of the subject matter described herein can include one or more non-transitory computer-readable devices having instructions stored thereon that, when executed by one or more processors, cause performance of operations comprising generating electrocardiogram results from the human; and comparing the results to a signal template to obtain an indication of the level of the analyte in the human, wherein the signal template was generated based on assessments of the level of the analyte within a population of humans other than the human from which the electrocardiogram results were generated.

Some implementations of the subject matter described herein can include one or more computers and one or more computer-readable media having instructions stored thereon that, when executed by the one or more computers, cause performance of operations comprising: obtaining data indicating electrocardiogram results from the human; identifying a plurality of beats represented in the electrocardiogram results; determining, for each beat in the plurality of beats represented in the electrocardiogram results, a value for a first feature of the beat; performing statistical analysis on the values for the first feature of the plurality of beats; generating an indication of the level of the analyte within the human based on a result of the statistical analysis performed on the values for the first feature of the plurality of beats; and providing the indication of the level of the analyte within the human.

Some implementations of the subject matter described herein can include one or more computers and one or more computer-readable media having instructions stored thereon that, when executed by the one or more computers, cause performance of operations comprising: generating electrocardiogram results from the human; and comparing the results to a signal template to obtain an indication of the level of the analyte in the human, wherein the signal template was generated based on assessments of the level of the analyte within a population of humans other than the human from which the electrocardiogram results were generated.

## BRIEF DESCRIPTION OF THE DRAWINGS

Fig. 1 is a plot that depicts an estimation of a patient's heart rate before QRS detection.

Fig. 2 is a plot of a wavelet response of an ECG signal with an adaptive threshold.

Fig. 3 is a plot of automatic scoring of an ECG for the detection of artifacts. The plot includes an initial ECG signal and several derived signals sued in detected an artifact in the initial ECG signal and making a decision to reject a segment of the signal containing the artifact.

Fig. 4 is a plot of the original ECG signal from Fig. 4, including a dashed-line box surrounding the portion of the ECG signal that has been marked for rejection as being overly noisy.

Figs. 5A-5C are plots that may be presented by a temporal analysis tool to depict a progression of ECG features over time, along with indications of calculated analyte levels, measured analyte levels, or both a corresponding times.

Figs. 6A-6B are plots showing T-right slope values over time.

Fig. 7 is a graph that plots two lines, namely (1) a pre-filtered plot of T-right slope values and (2) a filtered plot of T-right slope values smoothed by application of the Kalman filter.

Fig. 8 is a plot showing several beats from an original ECG signal. The original signal is superimposed with a plot of the ECG signal after processing used time principal component analysis (PCA) filtering techniques.

Figs. 9A-9C are plots that show estimations of the autocorrelation and probability distribution functions of the T-wave. Fig. 10A shows a signal averaged T-wave. Fig. 10B shows the probability distribution function of a T-wave. Fig. 10C shows a sample autocorrelation function of a T-wave.

Fig. 10 depicts histograms of T-right slope values determined from an electrogram data for different potassium levels.

Fig. 11 is a plot a plot in which a histogram of the T-amp feature has been fitted to both a normal probability distribution function and to a gamma probability distribution function.

Figs. 12A-12B show 2d image representations of a series of beats identified from electrogram data over time.

Figs. 13A-13B show 2d image representations of the series of beats from Figs. 14A-14B after applying local signal averaging.

Figs. 14A-14B show plots of the demodulation of a patient's respiratory (breathing) signal from the ECG signal.

Fig. 15 is a plot of an ECG signal to which an artifact detection filter has been applied. The plot shows a first zoomed region (left) of a clean segment of the ECG signal that is not rejected by the filter, and a second zoomed region (right) of a noisy segment of the ECG signal that is rejected by the filter.

Fig. 16 depicts a temporal progression snapshot for presenting long ECG segments using the techniques described herein for cleaning the signal from noise and artifacts, peak detection, features extraction, and features tracking.

Fig. 17 depicts noninvasive estimation of potassium levels vs. lab potassium values.

Fig. 18 depicts statistics of the error in estimation of the potassium when tested against blood potassium values.

Fig. 19 depicts trend detection of the potassium, including comparison of the estimated potassium vs. actual value over time.

FIG. 20 depicts three plots representing models of potassium concentrations vs. extracted waveform feature T- wave right slope ! Sqrt(T-wave peak amplitude). The first plot (A) is an initial model generated based on an initial set of data points. The second and third plots (B-C, respectively) are updated models that are refined as additional data points are added to the initial set over time.

FIG. 21 depicts an ECG processing system configured to process combinations of signals from one or more input sources to generate estimated analyte levels.

FIG. 22 depicts a plot of example input signal features that may be processed by a model to determine estimated analyte levels for a patient.

Fig. 23 is a block diagram of computing devices that may be used to implement the systems and methods described in this document.

Like reference numbers among the drawings indicate like elements.

## DETAILED DESCRIPTION

This document describes computer-based techniques for quantifying the concentration of analytes, such as potassium, in a patient's blood based on physiological electrical data (electrogram data). The physiological electrical data may be obtained using any suitable technique such as electrocardiogram ("ECG") measurements (which may include surface, intracardiac, or subcutaneous ECGs, or measurements obtained using a pacemaker implanted in a patient's body, or defibrillators, for example). Other physiological electrograms may also be employed, including brain electrograms ("EEG"), muscular electrograms, myoelectrograms that cover smooth and striated muscle, for example, and neuro-electrograms. Either or both tonic and resting physiologic electrograms may be employed, as well as electrograms that measure responses to provocations such as evoked stimuli or extrinsic electrical stimulation or other stimulation.

The systems, methods, and other techniques disclosed herein can be applied to detect physiological conditions of a subject. For example, a system can non-invasively measure and/or determine changes in analyte levels of the subject (e.g., levels of calcium, potassium, magnesium, glucose, and the like), determine levels and monitor the effects of pharmaceuticals (e.g., digoxin, Class I-C anti-arrhythmia drugs, sotalol, dofetilide, amiodarone, beta blockers, calcium channel blockers, tricyclic antidepressants, lithium, and the like), detect physiological abnormalities (e.g., acute myocardial infarction, subarachnoid bleed, fluid accumulation around the heart, and the like), or a combination of these. In some embodiments, the system can monitor for compliance with avoidance of drugs such as cocaine, amphetamines, marijuana, and the like.

In the context of this document, electrogram data generally refers to an electrical recording of any electrically active biological tissue, whether recorded from a traditional surface ECG electrode, custom body surface electrodes that may vary in size, shape, and inter-electrode distance, for example, or from intracoporeal electrodes, whether they be subcutaneous, intracardiac, or within other tissues or natural cavities. Electrograms from which such data is obtained may be spontaneous, or in response to a stimulus or provocation, and may be recorded from contact or non-contact electrodes. By way of example, the electrogram data may be obtained from one or more physiological electrograms including electrocardiograms (ECG), brain electrograms (EEG), muscular electrograms, myoelectrograms, and neuro-electrograms. In some examples, electrogram data may be obtained from one or more of a standard 12 lead ECG, a wearable patch with one or more channels, and wearable elements including shirts, watches, bands and bracelets with conductive elements capable of recording physiologic signals. Electrogram data may be obtained in some examples from implanted devices such as loop recorders, pacemakers, and/or defibrillators. In some implementations, ECG data may be collected during a dialysis session. The ECG data may be processed in real-time to monitor changes in electrolyte levels during dialysis. For example, every 10, 15, 30, 45, or 60 seconds, updated analyte levels may be determined based on the latest ECG data available. Such information may be used to adjust the duration or intensity of dialysis sessions. In this embodiment, the ECG signal may be acquired from skin worn electrodes, or from electrodes integrated into the dialysis fistula or the equipment used to access the fistula. Filters may be added to clean electrical signals by removing dialysis machine electrical noise.

While the term "computer-based" is applied, it is recognized that this may refer to any suitable form of computer processing, including mobile-based processing. For example, the techniques disclosed herein may be implemented at least in part by a mobile computing device such as a smartphone, tablet, or notebook computer that communicates with a system of wearable or hand-held electrodes. These techniques may also be implemented in wearable ECG patches or implantable devices. These techniques permit data compression and distribution of processing among various aspects of such a system, to enable near real-time, frequent, analyte assessment in ambulatory/outpatient individuals. This may be particularly useful in dialysis patients who are at risk for abnormal analyte levels (e.g., hyperkalemia), patients with cardiac disease, and/or renal insufficiency. This document discusses quantifying concentrations of potassium in some examples, although similar techniques may also be used to quantify concentrations of other analytes as well, including quantification of drug levels. Additionally, this paper broadly uses the term "patient" to generally include any person from whom electrogram data is obtained, regardless of their clinical status for example.

Some techniques for processing electrogram data to assess analyte levels in a patient are described elsewhere, including in PCT Publication WO2015/048514, which is incorporated herein by reference in its entirety. This document describes additional techniques that may allow for an improved ability to derive reliable analyte level assessments from noisy electrogram data, as tends to be more common in data recorded from home-use electrogram devices. Additionally, technqiues are described herein by which templates for analyte assessments may be derived from a population of patients and applied to a given patient not among the population, thereby obviating the need for blood tests from each individual patient.

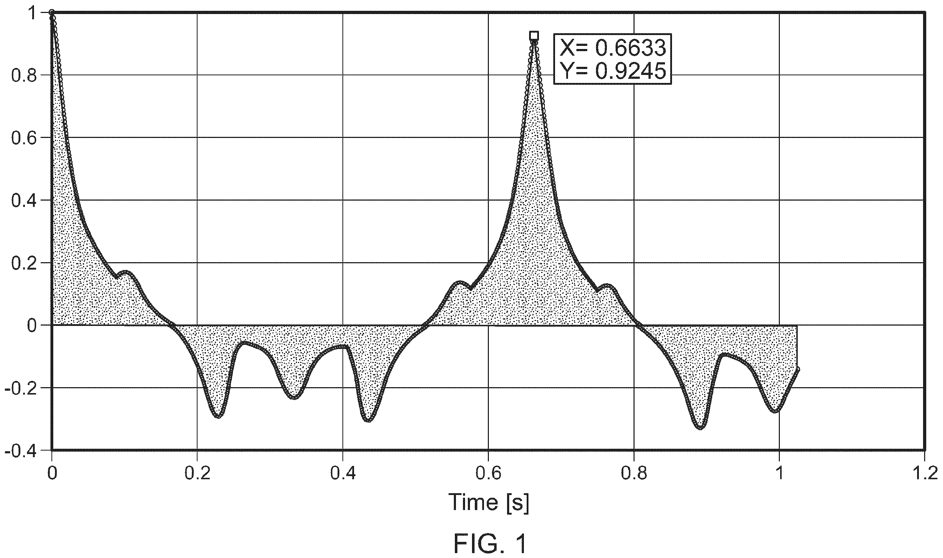

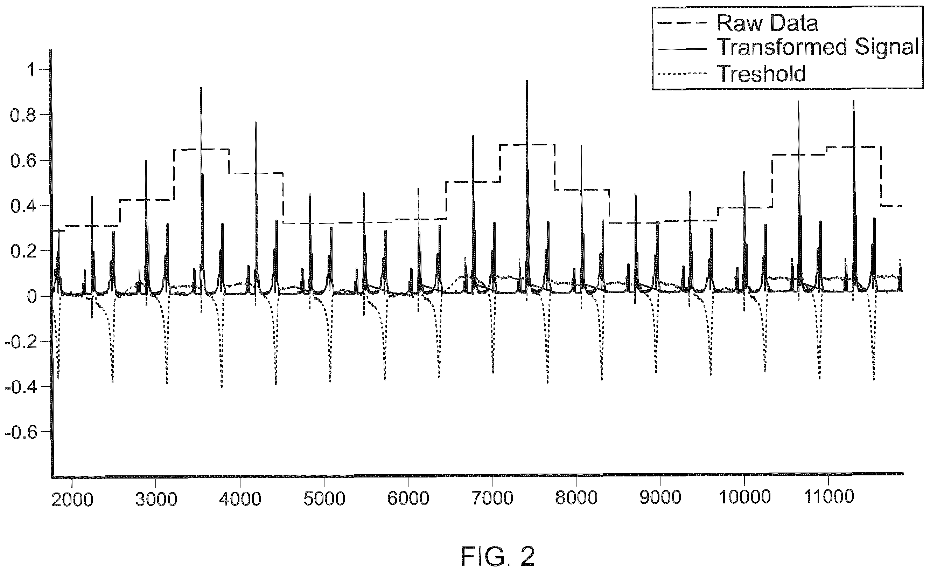

In some cases, the ECG data recorded from a patient may have relatively low signal to noise ratios, particularly when the data originates from a single-lead ECG as a patient might use at home. The noisy signal may make it more difficult to the detect QRS complexes, and to discern the QRS complexes from noise or other features in the ECG signal. In order to reliably detect QRS complexes from a noisy ECG signal, information about the patient's heart rate during the ECG may be used to more accurately focus in the relevant band of frequencies when performing QRS detection. For example, a continuous wavelet transform with the "Haar" wavelet may be performed on the ECG signal to translate the signal from the time domain to frequency domain. In some implementations, other types of transforms may also be utilized, such as a discrete wavelet transform with the "Haar" wavelet or other wavelets, a Daubechies transform, a Meyer transform, or a Symlet transform. The QRS complexes are identified as the peaks of the wavelet transform that are greater than a threshold value within a certain frequency range. The threshold may be adaptive and set the local maximum for a window set for the estimate R-R interval (the time between successive QRS complexes, which relates to a patient's heart rate). The R-R interval estimation can be performed determining, from the autocorrelation function R(t) of the ECG signal, the highest positive peak in the autocorrelation function between 200 ms (200 bpm) to 1500 ms (40 bpm). The estimated R-R interval can then be applied to the analysis of the wavelet transform of the ECG signal to focus only on candidate QRS complexes (peaks in the QRS transform) within a limited band of frequencies above and/or below the frequency indicated by the R-R interval. Each candidate QRS complex can then be compared to correlate the median optional QRS, and from this abnormal QRS may be discarded. In this way, QRS complexes may be more reliably identified even in noisy signals, and may reduce the likelihood of very sharp T waves being mistakenly identified as a QRS complex, for example. Aspects of these techniques are illustrated, for example, in Figs. 1 and 2. In particular, Fig. 1 is a chart that plots an estimation of a patient's heartrate before QRS detection, and Fig. 2 is a plot of a wavelet response of the ECG to which an adaptive threshold has been applied.

In some implementations, the computing system processing ECG data can detect and reject artifacts in the data that may result from factors such as external noise, noise from the patient's body, mislocated lead(s), and bad contact between the lead and surface of the patient's body. Especially when data from home-use ECG recorders having few leads (e.g., a single lead) are used, the ECG signal may have a relatively low signal-to-noise ratio as compared to a standard 12-lead ECG setup. Therefore, in some approaches, overly noisy data can be rejected and discarded from further use in processing the ECG data to make analyte assessments or arrhythmia predictions. Various signal processing techniques may be used for artifact detection, which may include an analysis of the ECG data itself, and which may optionally an analysis of external signals other than the ECG data as well. One example of such an external signal includes a signal from one or more accelerometers. The accelerometer signal can be analyzed to determine whether the patient is moving and the nature of any such movement. Noise and other artifacts in the ECG signal are more likely to occur while the patient is in motion (e.g., changes positions, walks, twitches). Therefore, the accelerometer data may be synchronized to the ECG data so that segments of the ECG data that correspond to times when the patient was in motion can be identified. These segments may be automatically discarded under a presumption that artifacts are likely to be present during times of patient motion, or the segments may be automatically selected or flagged for further analysis for artifact detection according to the techniques described herein. Accelerometer data, or data from other non-ECG sensors, may also be used to determine when to record ECG data (e.g., choose to record ECG data while the patient motion is below a threshold level, but choose not to record while the patient motion is above a threshold level). Accelerometer data may be used in some examples to determine when the patient's body is in an appropriate position/orientation to acquire quality signals. For example, if the accelerometer data recorded from accelerometers worn by the patient are determined to match a template that indicates the patient is in a proper position/orientation, then the computing device may automatically alert a user that the patient is in the proper position/orientation and/or may automatically begin acquiring the ECG data. In some implementations, accelerometer data and/or data collected by a respiratory belt worn by the patient may be processed and used to remove noise, artifacts, and the like from an ECG signal or for additional feature extraction. The additional feature can be used for selecting segments in the ECG for analysis or for adjusting the ECG extracted features for potassium-level estimation (or other analyte-level estimation). In some examples, segments of the ECG signal that were selected using the accelerometer data can be used to find a transformation between patients' different body positions.

In some implementations, this artifact detection and rejection approach can be used in lieu of a signal-averaging approach in which the ECG signal is filtered to derive a cleaner averaged representation of a heartbeat.

In some implementations, the artifact detection and rejection approach can be used in conjunction with the signal-averaging approach. The choice of whether to apply signal-averaging, artifact detection and rejection, or both may be selected by a user (e.g., by a qualified healthcare professional), or may be automatically determined by the computer processing system based on the context of a given setup (e.g., whether the patient is connected to a standard 12-lead ECG or a single-lead ECG) or based on characteristics of the recorded data. In some implementations neither signal averaging nor artifact rejection are applied, and a statistical or template-based approach is used to analyze the physiologic signals.

Generally, the computing system can detect artifacts and remove segments of the ECG signal containing those artifacts to create a reliable and clean ECG beat. The computing system can use linear filtering techniques, non-linear filtering techniques, or both to detect artifacts that are to be discarded. For example, sudden changes in the ECG low frequencies and/or baseline due to strong noise or baseline drift can be detected and rejected from the signal. In some implementations, the artifacts can be scored and compared to a threshold value. Artifacts having a score that satisfies the threshold may be discarded, while artifacts having a score that does not satisfy the threshold may be retained in the signal for further processing. The threshold may be static and pre-determined, or it may be an adaptive threshold that, for example, accounts for a limit on the amount of discarded data that is permitted to be discarded. Thus, the threshold may be lowered as the discarded data limit is raised, whereas the threshold may be raised as the discarded data limit is lowered.

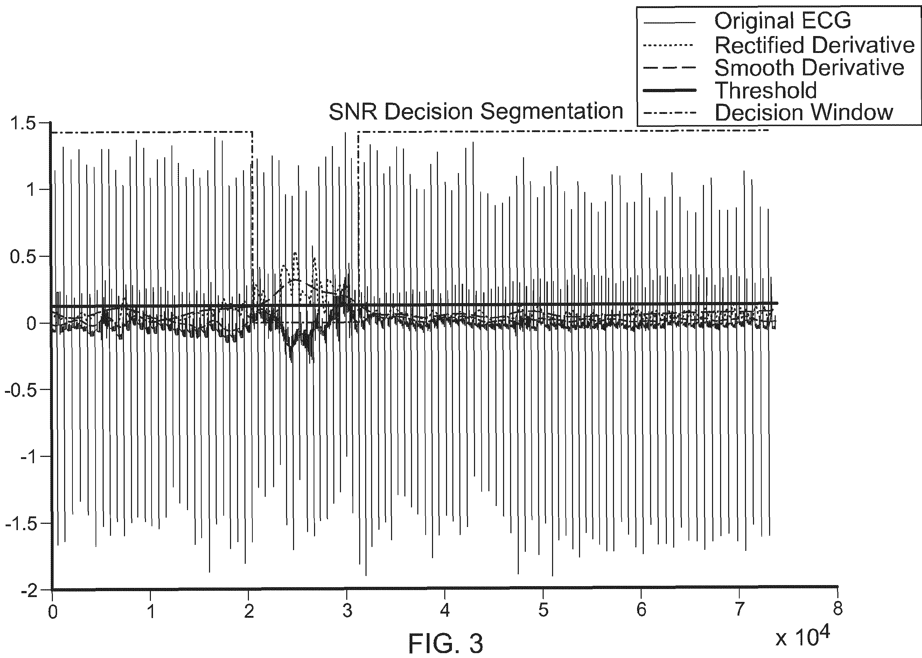

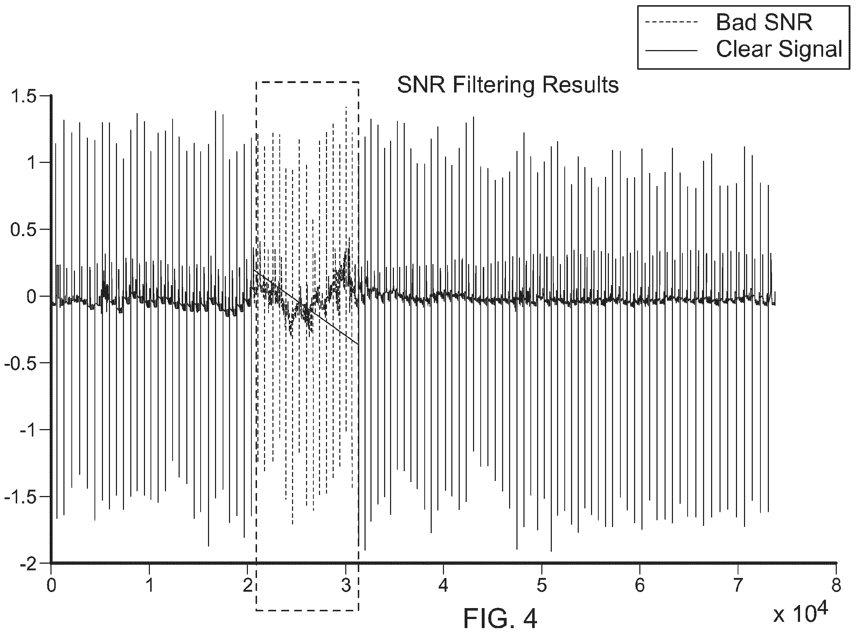

Figs. 3 and 4 illustrate one example of artifact detection and rejection techniques used to clean an ECG signal. Fig. 3 is a plot of an original ECG signal and several derived signals used in detecting an artifact in the original signal and making a decision to reject a segment of the signal containing the artifact. In particular, a threshold value is shown by the horizontal line extending across the plot. A smoothed derivative of the ECG signal is also plotted. A decision is made to reject the segment of the signal anywhere the smoothed derivative plot falls above the threshold value. In some implementations, the smoothed derivative may indicate a level of noise in the signal at any given time (e.g., SNR or inverse SNR), such that when the noise level exceeds the threshold value, the corresponding segment of the ECG signal is identified as an artifact and marked for rejection from the signal. Fig. 4 is a plot of the original ECG signal from Fig. 3, including a dashed-line box surrounding the portion of the ECG signal that has been marked for rejection as being overly noisy. In some implementations, in addition to ECG-based threshold values, accelerometer-based thresholds are used to reject data collected during periods of excessive (threshold-exceeding) mechanical motion.

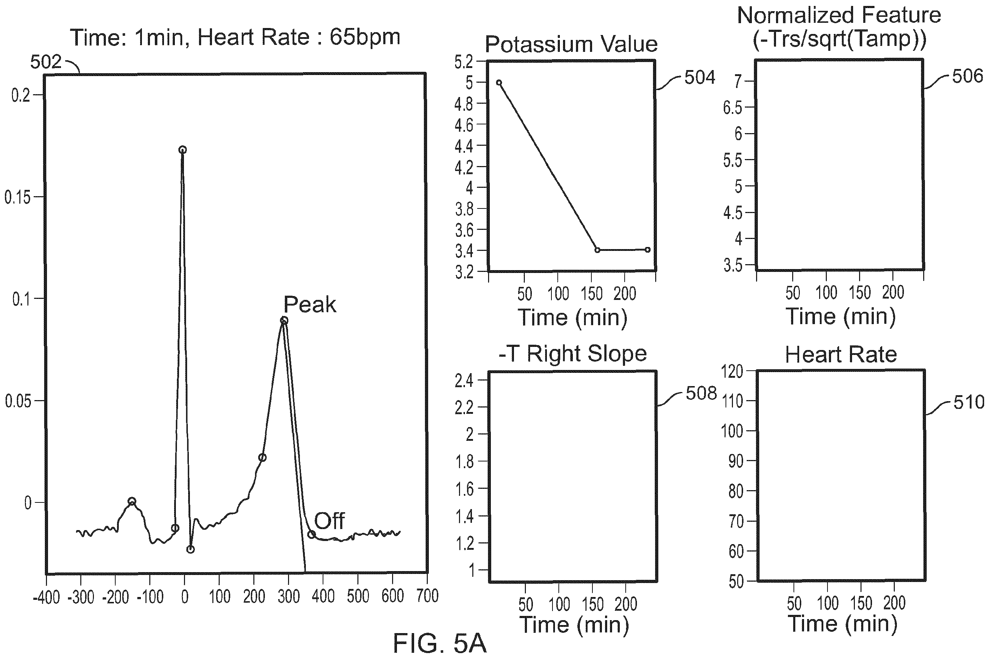

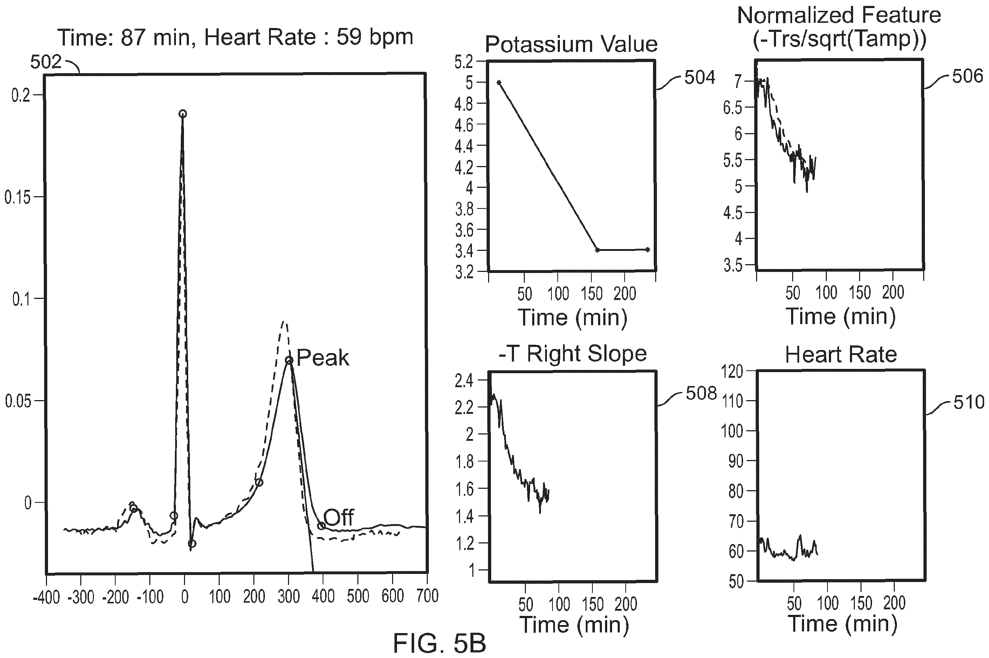

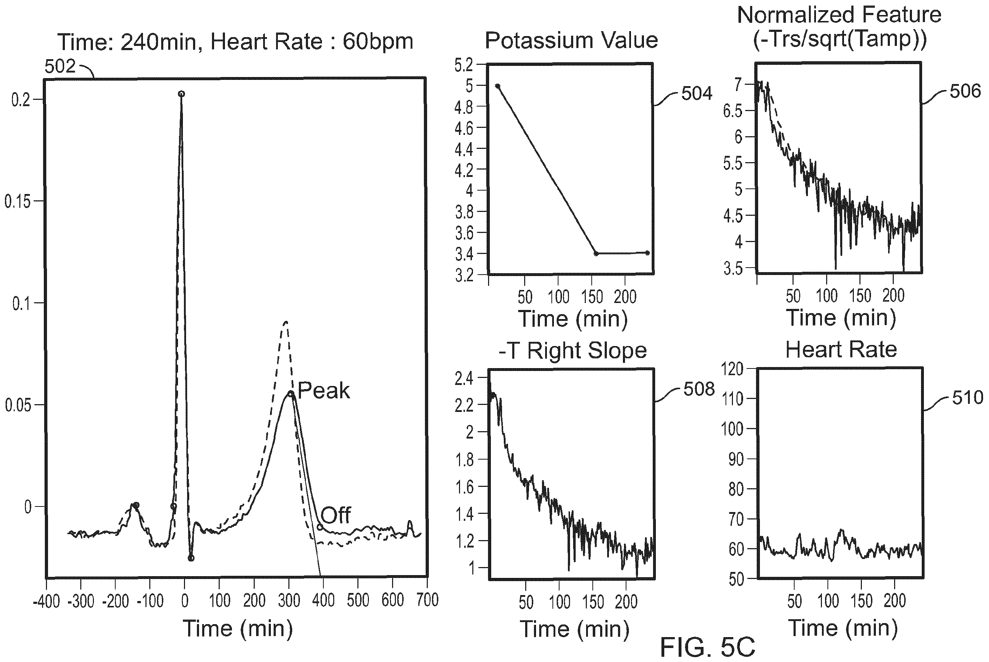

In some implementations, the computing system includes a temporal analysis tool that is operable to generate graphical representations of a processed ECG signal. The tool may depict a progression of ECG features over time, along with indications of calculated analyte levels, measured analyte levels, or both at corresponding times. One example of such a graphical representation is shown in Figs. 5A-5C. These figures include several plots that may be simultaneously displayed by a computing device, such as within a given application or application window. The display may include different combinations of the plots, which may be selected according to default settings or may be specifically selected by a user for a custom display. In the example of Figs. 5A-5C, an application window having five plots is shown at different points in time during a dialysis session for a patient. The plots in Fig. 5A are from a time at the start of the dialysis session, whereas the plots in Fig. 5B are from a time around a midpoint of the dialysis session, and the plots in Fig. 5C are from a time near the completion of the dialysis session. While these few points in time are shown by way of example, in practice, the temporal analysis tool may show a progression at regular intervals over the dialysis session or during any time interval during which potassium or other analyte levels may vary. At each time interval, the plots may be automatically updated to show the corresponding data for the new time. In some implementations, the temporal analysis tool may create a video file or animation that shows the progression of the patient's ECG and analyte parameters over a period of time (e.g., during an entire dialysis session). For example, the processed ECG signal may be spliced into small, overlapping segments using signal averaging techniques (and/or artifact detection and rejection techniques). The temporal analysis tool may then plot the relevant features for each time segmented portion of the dialysis session. In one example, the ECG may be segmented into one minute intervals so that the plots are updated for each one minute interval, although the video may be speeded up to more readily see the changes in data over time, such that each second the plots are updated to show the data processed from the next one-minute interval. In this way, a progression of one-minute segments for a 4-hour dialysis session may be viewed relatively quickly (e.g., 2 minutes or less). In some implementations, the segmented ECG data may include overlapping portions of data from adjacent segments. For example, a first segment calculated for the two-minute mark of the dialysis session may include data from the preceding 35-seconds of dialysis and data from the following 35-seconds of dialysis. A second segment calculated for the three-minute mark of the dialysis session may likewise include data from the preceding 35-seconds of dialysis and data from the following 35-seconds of dialysis, so that there is overlap in the data used in both the first segment and the subsequent, second segment. The segment interval and the amount of overlap may be set by default or may be set as a result of user input to the computing system.

In each of Figs. 5A-5C, plot 502 shows a beat from the processed ECG along with a baseline sample ECG beat. In some implementations, the baseline sample ECG beat may be the processed ECG from a starting time of the dialysis session. Thus, in Fig. 5A, which indicates a segment from the start of the dialysis session, the baseline sample ECG and the segment-specific processed ECG are identical. However, in later segments as shown in Figs. 5B and 5C, the segment-specific plot of the processed ECG diverges from the baseline sample so that the change in the ECG beats over time are readily apparent. The plots 502 each also show the algorithmically calculated T-wave peak and offset, and a tangent line that is useful in assessing analyte levels from ECG data. The plots 504 show true potassium levels measured in the patient's bloodstream at certain times before, during, and upon completion of the dialysis session. The plots 506 show normalized features, both before and after application of a Kalman filter, over time. The plots 508 show the t-right slope from the beats at each segment, plotted over time. The plots 510 show the patient's heart rate at each segmented interval, plotted over time.

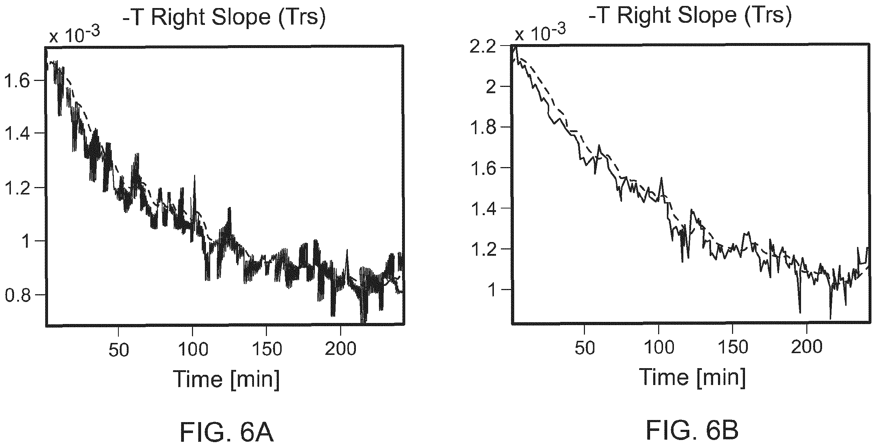

As has been described elsewhere, the T-right slope value determined from a processed ECG signal can be used in assessing a patient's analyte level. Some techniques for determining T-right slope involve determining from the slope from a starting point at the peak of the T-wave to an ending point at the end of the T-wave. However, in noisy signals, it is often difficult to pinpoint the T-wave's peak and ending points. Therefore, in some implementations, the computing system may calculate T-right slope by selecting points in the range between the peak and ending points. Points that define a mid-part of the wave may thus be used to calculate T-right slope, as the mid-part of the wave generally has a substantially constant slope and can be more reliably used to estimate the entire slope than other techniques. By deriving slope from a mid-part of the wave, the need to determine the end of the T-wave can be avoided. Figs. 6A and 6B show plots of T-right slope values over time.

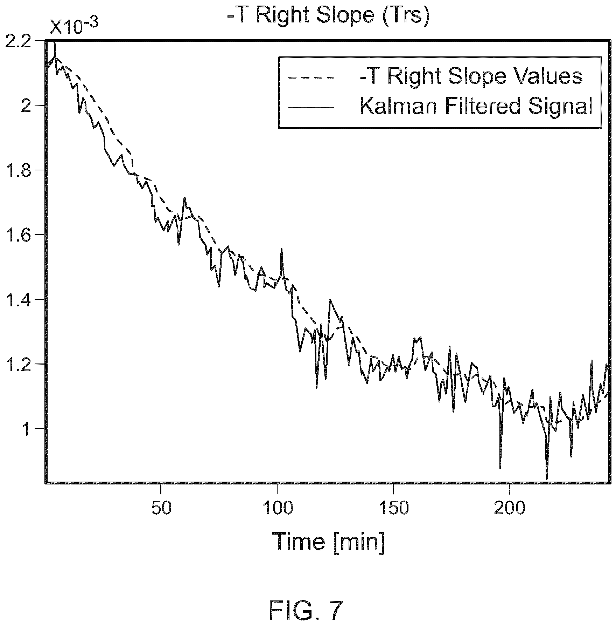

In some implementations, features from a processed ECG signal may be smoothed by use of a filter. For example, potassium levels in a patient are not expected to exhibit significant changes over very short periods of time. Potassium levels instead tend to change relatively slowly and gradually over a period of time. With this understanding, it is reasonable for the computing system to apply smoothing to ECG features used in determining potassium levels. The raw ECG data may be quite noisy and exhibit sharp changes over short periods of time, even as the trend of the ECG data over a longer period of time indicates a more gradually changing characteristic. Therefore, the computing system may apply a filter to all or portions of features from an ECG signal, so as to mitigate noise and produce a smoother of the feature (e.g., a T-right slope feature). In some implementations, a Kalman filter (also referred to as "linear quadratic estimation") may be applied to smooth the ECG signal. Figure 7, for example, is a graph that plots two lines, namely (1) a pre-filtered plot of T-right slope values and (2) a filtered plot of T-right slope values smoothed by application of the Kalman filter. The ultimate determination of potassium levels (or levels of other analytes, as appropriate), may be determined based on the filtered T-right slope values.

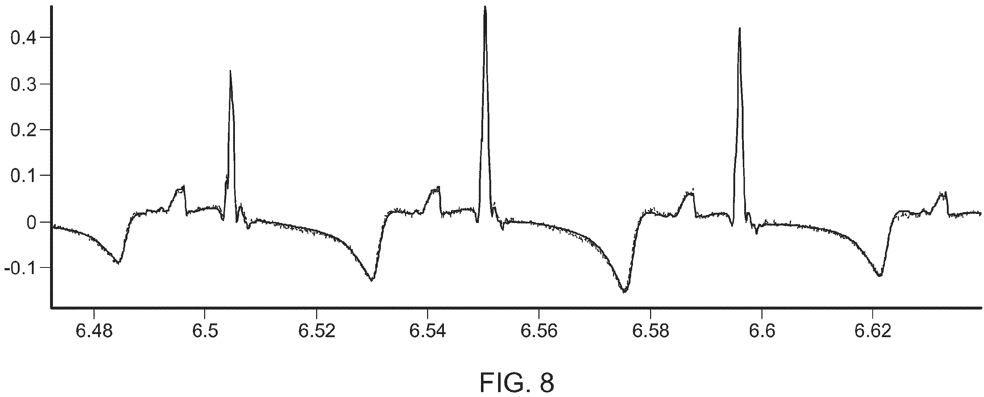

Referring to Figure 8, a plot is shown of several beats from an original ECG signal. The original signal is superimposed with a plot of the ECG signal after processing used time principal component analysis (PCA) filtering techniques. In some implementations, the ECG signal can be processed using Limited Karuhan-Loeve expansion, which can facilitate extraction of key signal components. These techniques are useful to reduce the noise in the signal. Generally, PCA filtering involves Eigen value decomposition and separation between strong eigenvectors known as the signal. Among the key signal components that may be extracted using PCA filtering are representing beat and the main noise factor. PCA known as the Karuhan-Loeve Transform (KLT) can be used for noise reduction using a limited version of the original KLT column.

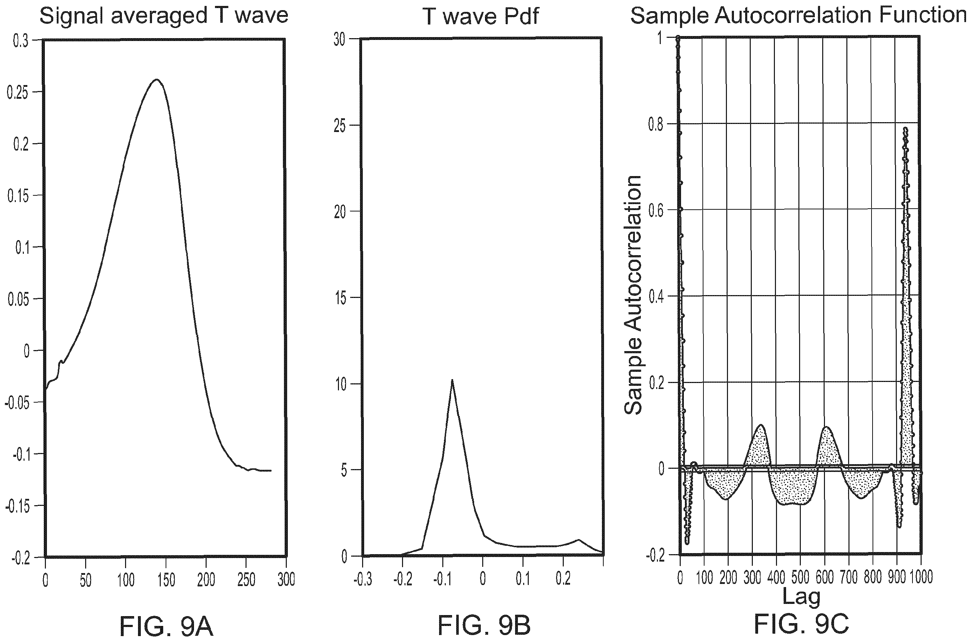

In some implementations, the computing system may correlate different potassium levels (or levels of other analytes) with changes in the statistics of the ECT/ T wave. Referring to Figures 9A-9C, plots are shown of histograms of the T wave as an approximation of the t wave data's density function and the second moment function (autocorrelation).

In some implementations, the computing system may select one or a subset of all the available leads from which ECG data is collected to analyze as the basis for determining analyte levels in a patient. When ECG electrode patches are attached to a patient, there is often variance in the relative location of electrodes in the patch on the patient as compared to their locations on the patient in previous ECG sessions. Differences in the locations at which electrodes are placed on the patient can impact characteristics of the recorded ECG signal. To mitigate differences resulting from patch location variances, the computing system may select data from a particular lead for analysis, and may discard data from one or more other leads. The lead chosen for analysis may be selected, in some implementations, based on comparison of the recorded ECG signal (or features of the recorded ECG signal) to a pre-stored signal template (or comparison to one or more feature templates). The data from each available ECG lead may be compared to the template(s) and a similarity score determined for the data from each lead using, for example, pattern recognition techniques. The data for a given lead having the highest similarity score may then be selected for further processing and analysis in the analyte assessment to the exclusion of data for the other leads. In some implementations, the signal template is generated based on QRS Complex morphology. Some pattern recognition techniques that may be employed in comparing the recorded ECG data to the template(s) include principal component analysis ("PCA") and spanning the function using the coefficients results in the best approximation to the QRS complex.

In some implementations, the computing system may assess a patient's analyte levels based on a statistical analysis of features in a processed ECG signal. Elsewhere, signal averaging techniques have been described for assessing analyte levels in a patient's bloodstream. Generally, signal averaging techniques can involve averaging ECG data for multiple beats over a period of time to create a representative beat. The representative beat is then analyzed to determine its relevant features (e.g., T-right slope, T-peak value), and the analyte level is then determined based on the features from the averaged, representative beat.

In some implementations, other statistical techniques may be employed in conjunction with or alternatively to signal-averaging. In one approach, the computing system determines feature values (e.g., T-right slope, T-peak value, T-wave shape) for individual beats in the ECG data (rather than for an averaged beat derived from an average of multiple beats). Feature values may be calculated for every beat, or for a subset of beats over a period of time, such as if certain beats have been discarded due to the presence of excessive signal noise. Once the feature values have been determined for each of the applicable beats over a period of time, statistical techniques may be used to select the ultimate feature value(s) from which the computing system calculates the estimate of analyte levels in the patient's bloodstream for that period of time. For example, the mean or median T-right slope value of all the beats over a period of time may be discerned and applied in the analyte level calculation. Other higher-order statistics may be used in some implementations to select the ultimate feature value(s) from which the analyte levels are determined, such as variances, skewness, and kurtosis.

In some implementations, the computing system performs statistical analysis on raw values of the ECG data for individual beats. The system can use statistical tecniques to select raw values of the ECG data that correlate with estimated analyte levels in the patient's bloodstream. For example, the mean or median of the raw ECG values for one or more beats, or portions thereof, may be computed and applied in the analyte level calculation. Other higher-order statistics may be used in some implementations to select the raw ECG value(s) from which the analyte levels are determined, such as variances, skewness, and kurtosis. In some implementations, the system combines statistical analysis on raw values of the ECG data and feature values for individual beats derived from the ECG data to select the criteria, e.g., feature value(s) or raw waveform values, from which to determine analyte levels, such as variances, skewness, and kurtosis of the feature value(s), raw waveform values, or both.

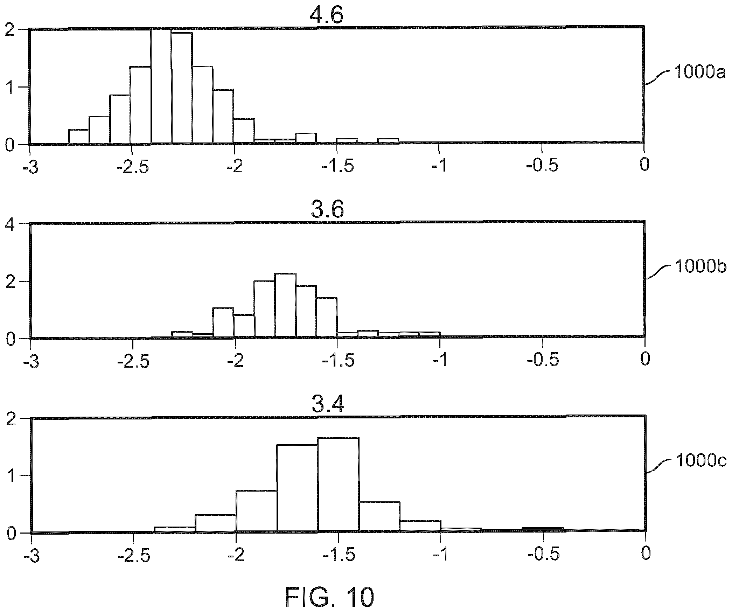

In some implementations, the computing system generates histograms of feature values from multiple beats over a period of time to facilitate a statistical analysis of beats from recorded ECG data. For example, Fig. 10 shows three histograms 1000a, 1000b, and 1000c of T-right slope values from a patient. Each histogram indicates the T-right slope statistics at a different potassium level. In particular, plot 1000a is a histogram of T-right slope values at a potassium level of 4.6 Mmol/L; plot 1000b is a histogram of T-right slope values at a potassium level of 3.6 Mmol/L; and plot 1000c is a histogram of T-right slope values at a potassium level of 3.4 Mmol/L.

In some implementations, the system assigns to each beat in a set of beats characterized by ECG data a respective quality score that indicates a quality of the recorded beat, and hence a suitability of the beat for processing. The system may compare the quality score for each beat to a static or dynamic threshold score and, based on the comparison, take appropriate action with respect to the beat. For example, if the quality score is too low, i.e., indicates insufficient quality, then the beat may be discarded. If the beat is discarded, it may be excluded from a set of beats that are averaged to form a representative beat using averaging techniques. In other implementations, the features of the beat may be excluded from a set of beats from which features are derived and used in a statistical analysis to determine a correlation between waveform features of beats and analyte levels. In some implementations, the system can evaluate the robustness of either an averaged representative beat or waveform features that have been statistically correlated with analyte levels based on the quality scores of the beats from which the averaged representative beat or waveform features were derived. A representative beat or correlated waveform features that were derived from higher quality beats may be more robust than a representative beat or correlated waveform feature derived from lower quality beats.

The quality score for a beat can, in some implementations be based on features or characteristics of the beat. In some implementations, the quality score is determined by fitting a portion of the beat's waveform from the J point to the end of the beat to a Gaussian curve. The score is then computed as the R-squared value of the fit. In some implementations, the quality score is determined as the ratio of the area of the T-wave before rectification and the area of the T-wave after rectification. In some implementations, the quality score is by comparing statistics for features of the T-wave to pre-defined patterns of T-wave features from the same species, e.g., a T-wave dictionary.

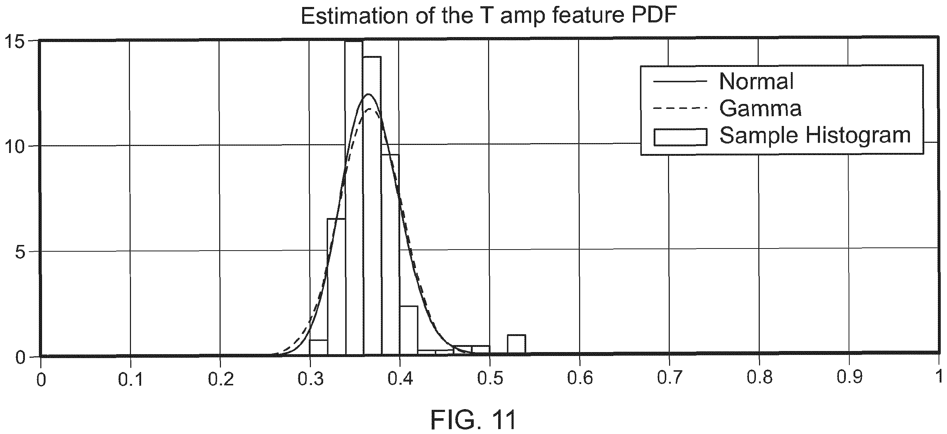

In some implementations, the computing system may use linear correlation techniques, non-linear correlation techniques, or both, to compare a morphologic pattern of the T-wave to potassium values. For example, Fig. 11 shows a plot in which a histogram of the T-amp feature has been fitted to both a normal probability distribution function and to a gamma probability distribution function. The parameters of the distribution functions may be determined, for example, using maximum-likelihood estimation. Higher-order statistics may then be performed based on the fitted distributions.

In some implementations, the computing system may compensate for different body positions when calculating the level of analyte in the patient's body. ECG signals are often sensitive to the patient's body position (e.g., whether the patient is standing or sitting when the ECG data is recorded). Therefore, different feature values may be derived from a patient's ECG data depending on the patient's body position at the time the data was recorded. In some implementations, to compensate for these differences, the computing system may store different templates for comparison against the recorded ECG data. Each of the stored templates may correspond to a particular body position and the recorded ECG data can be compared against an appropriate template for the body position.

By learning different feature values in different body positions (e.g., sitting, supine, tilted) for the same analyte level (e.g., the same K value ! potassium level), using one or more leads (together or separately), the computing system can transform features from a given set of ECG data to the features space in the position that the learning of the potassium curve was done. For example, a patient may wear a remote monitoring ECG that includes an accelerometer. By identifying two different known body positions within a short period of time, the potassium (or other analyte) level may be estimated using the ECGs captured at the corresponding times for each position. If the calculated potassium values for different positions are similar, then the values may be averaged and returned. On the other hand, if the potassium values differ by more than a threshold value, the measurement may be deemed invalid.

In some implementations, the computing system may remove noise from a set of ECG data before statistically analyzing the features of beats represented in the ECG data. To remove noise, the computing system may perform local signal averaging. The local signal averaging techniques can include aligning a plurality of beats by fiducial points in a 2d-matrix and applying an image processing filter (e.g., 2d-window filter) to smooth each beat with its neighboring beats over a relatively small window in time. In some implementations, a Gaussian filter may be used for the smoothing, although other types of filters are also contemplated.

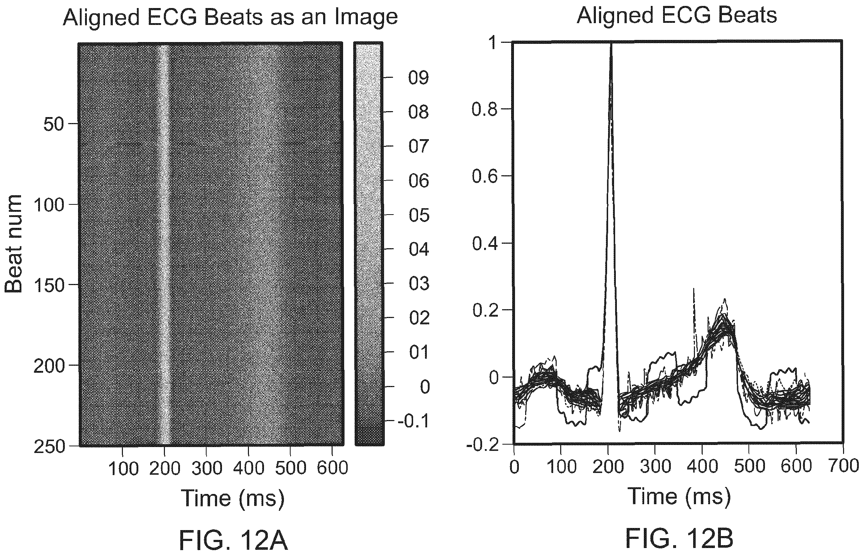

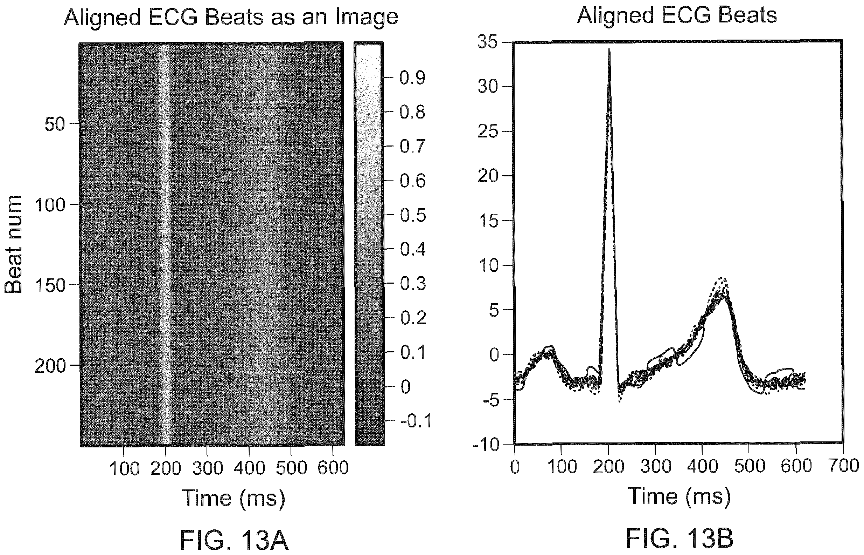

In some implementations, the computing system may generate image representations of beats from ECG data to facilitate visual analysis of the relations between neighboring beats. For example, Figures 12A and 13A each show the 2d images of a series of beats over a period of time. The images each include 250 rows, where each row corresponds to a different beat form a set of ECG data. The color / intensity of the image at each time (as indicated along the x-axis) for each beat indicates the amplitude of the ECG signal at that time. For a regular heartbeat, the distribution of colors over time for each beat represented in the image should be fairly similar. However, if the QRS complex is wider for some beats than their neighboring beats, for example, such an abnormality may be visually discernible from the image. Figures 12B and 13B are time-domain plots of a plurality of neighboring beats that have been aligned at one or more fiducial points (e.g., the peak of the QRS complex). Figures 12A and 12B depict representations of the ECG signals before local signal averaging was applied. Figures 13A and 13B depict representations of the ECG signals after applying local signal averaging.

In some implementations, machine-learning techniques can be used to build a model (e.g., a feedforward neural network, a deep convolutional neural network, a support vector machine, a Gaussian mixture model, a hidden Markov model, Bayesian decision rules, logistic regression, nearest neighbor model, decision trees, or other classifier models) that estimates analyte levels for a patient based on raw or pre-processed ECG data. In some implementations, the model may be trained to classify ECG segments to different analyte levels or bins (e.g., "low," "normal," "high" or "3-3.5," "3.5-4," "4-4.5"). Semi-supervised learning techniques can be employed in some implementations, which use labeled training data in which each sample of training data is pre-labeled with one of the possible output/classification values (e.g., "low," "normal," "high" or "3-3.5," "3.5-4," "4-4.5"). Each training sample may be, for example, data representative of a segment of ECG data. In some implementations, the training samples may be pre-processed to remove noise and smooth the signal. However, using established machine-learning techniques, the model can automatically discern patterns and features of an ECG segment that indicate the likelihood that the segment falls within of one or more classifications for a given ECG segment. If the model has a softmax output layer, for example, then a given ECG segment may be automatically classified into a most probable classification among the available classifications (e.g., "low," "normal," "high" or "3-3.5," "3.5-4," "4-4.5"). In some implementations, the model may classify based on features such as morphologic feature of mean beat and/or median beat, an estimated probability distribution function of the morphologic features, and classification of the ECG representation as an image.

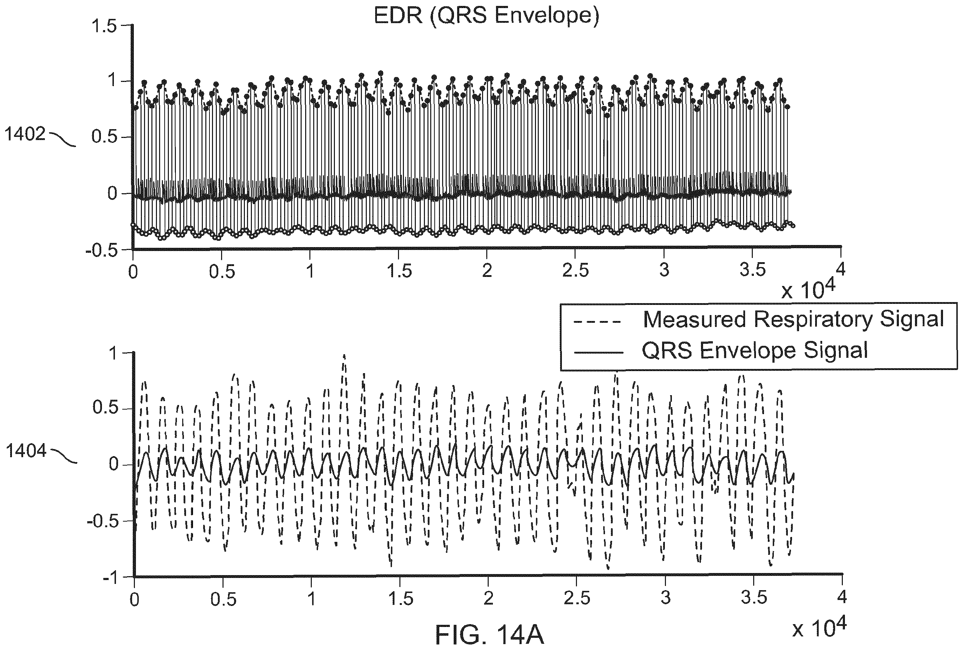

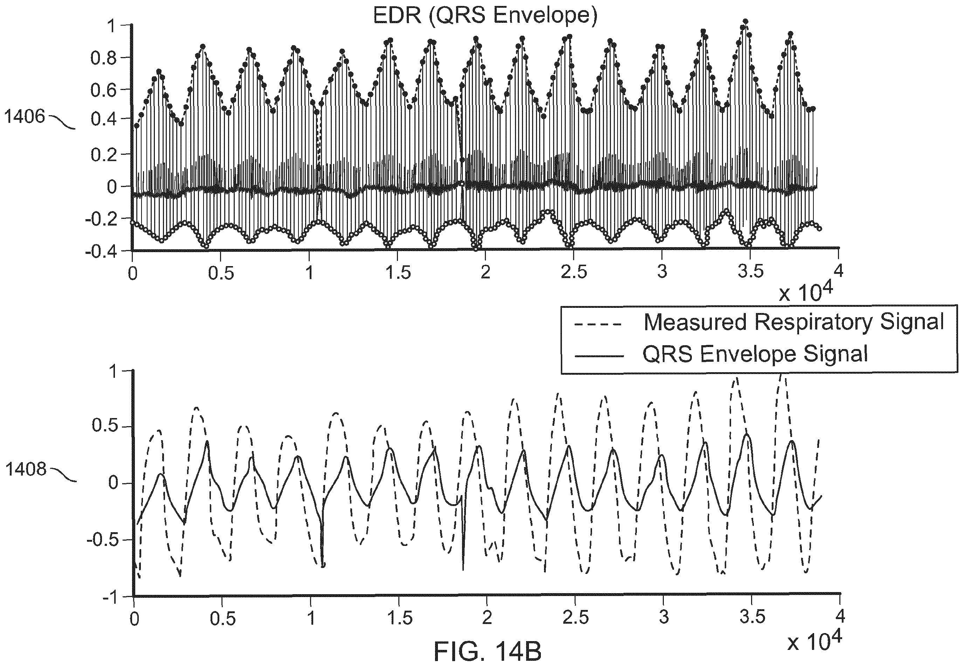

In some implementations, the computing system may process ECG data so as to substantially remove the impact of the patient's respiration on the ECG data. To this end, the computing system may derive the patient's respiratory pattern from the ECG using ECG derived respiratory techniques. Figures 14A and 14B show two examples of ECGs (in the top plot). The top envelope of the ECG signal generally include the peaks of QRS complexes across a series of beats, and the envelope generally correlates to the patient's respiratory cycle as indicated in the bottom plots of each of the figures. In some implementations, the computing system may analyze the QRS envelope from the ECG signal and take action to compensate for the impact of the respiratory cycle on the ECG signals. For example, the computing system may demodulate the envelope in some implementations before determining feature values and performing statistical analysis on the ECG signal.

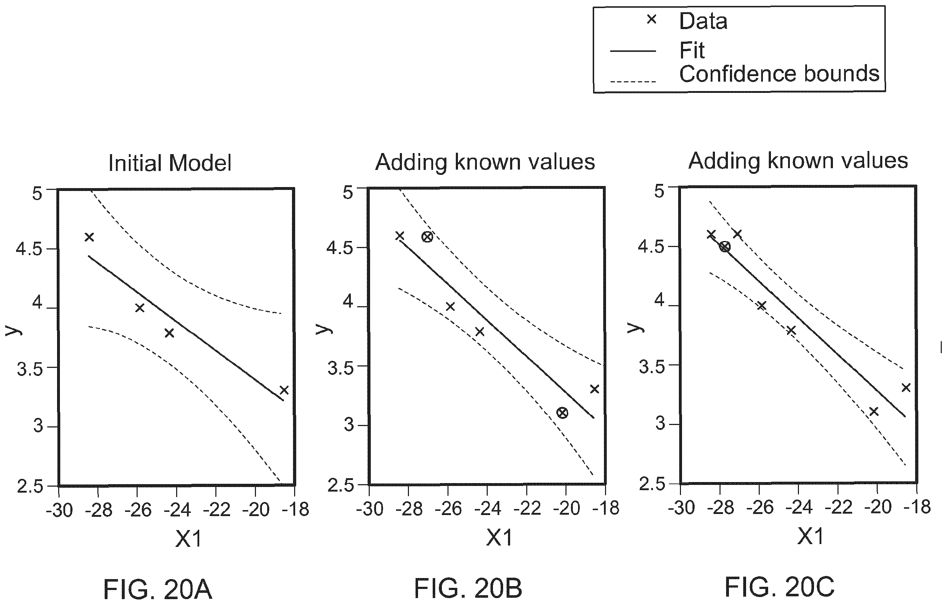

In some implementations, a personalized or population-based analyte estimation model may be constantly updated each time new, verified data points become available. The model may thus learn from new data and may evolve over time to generate predicted analyte levels based on ECG features with greater statistical confidence. For example, as depicted in Figure 20, in plot A, an initial linear model is fitted to an initial set of four data points. The initial data points indicate measured analyte levels in the patient's bloodstream (e.g., measured by a blood test) and the T-wave right slope/Sqrt(Tamp) value from an ECG at or near the time of the analyte measurement. The initial model may be used by some time for a patient, but as can be seen, the statistical confidence of estimated analyte levels is relatively low (as indicated by the separation between the model and the plotted confidence bounds). Subsequently, the patient may obtain additional verified data points that map measured analyte levels to T-right slope or other waveform features. The additional data points can be added to the model to refine and update the model. For example, as shown in Figure 20, plot B includes two new data points (indicated by the circled crosses), and the gap between the confidence bounds has narrowed as a result. Further, in plot B, a third new data point is added to the model at a later time, and the confidence bounds are shown to narrow even further. In some implementations, older data points may be removed as new ones are added to reflect potential changes in time and health status of the patient.

In some implementations, a computing system may provide a graphical user interface displayed on a screen of a computing system with which a user can interact to invoke a model update procedure. For example, the user may input the measured analyte values into the computing system via the user interface. Upon receiving the analyte values and identifying the appropriate ECG features for the times associated with the measured analyte values, the computing system automatically determines whether to update the model based on one or more criteria. For example, the system may determine whether the measured values are outliers that fall outside of a statistically acceptable range of values. In such cases, the system may reject the new data and block the model from being updated based on the new data. If the new data is within the acceptable range, the system may update the model automatically. In some instances, the computing system may maintain multiple models. The system may classify new data as being relevant to one or more of the models, and may select to update only those models for which the new data is classified as being relevant to. For example, the system may maintain models for each of a plurality of patient poses or positions. The system may identify (e.g., via user input or based on analysis of data from an accelerometer coupled to the patient) a particular pose or position of the patient that applies to a new data point and may select to update only one or more of the models that correspond to the identified pose or position. In some implementations, a user may use a mobile computing device (e.g., a smartphone or tablet computer) having a camera to take a photograph of test results. Software on the mobile device or executed on a remote cloud-based server may than analyze the photograph, extract the test results, and enter the extracted results into a database that can be used for updating models.

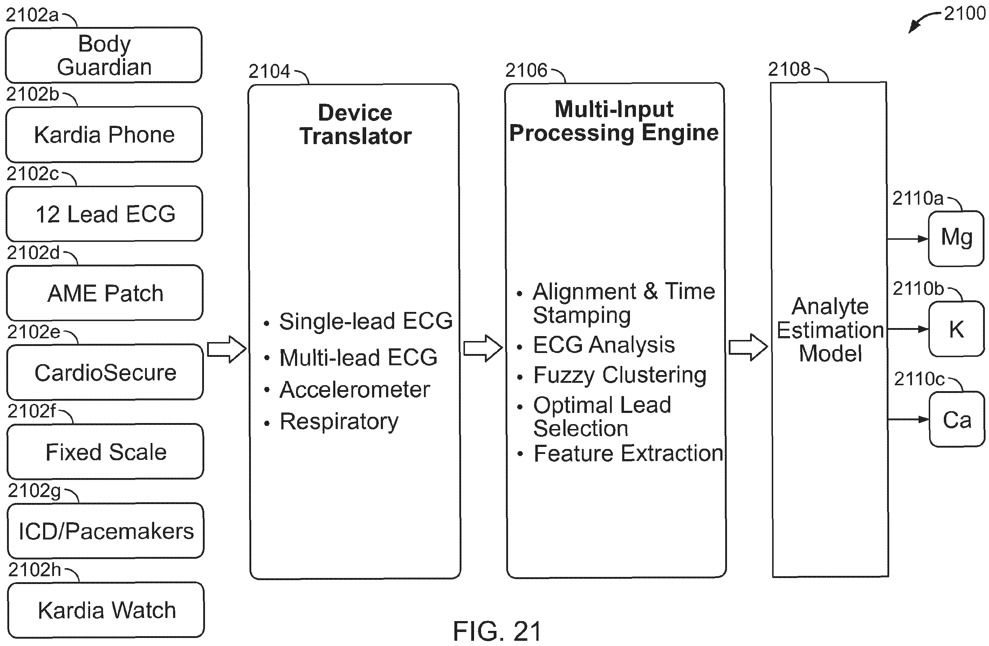

In general, an ECG processing system may be configured to process signals from multiple input sources. Information derived from these signals may be combined and applied to determine an estimated level of an analyte in a patient. To this end, Figure 21 depicts an example system 2100 that accepts multiple inputs to assess concentrations of analytes in a patient. In particular, the system 2100 is configured to perform batch or real-time processing of ECG data obtained from a set of input sources 2102a-h. In the example of Figure 21, various ECG sensing devices are deployed as input sources 2102a-h. Each of these devices is capable of generating ECG signals representative of a patient's cardiac electrical activity in different ways and in different settings. Depending on a patient's needs or preferences, he or she may tend to use different ones of the devices in different settings or situations. For example, a standard 12-lead ECG (2102c) may be preferable to obtain comprehensive results for evaluation of a patient in a clinical setting, but a BODY GUARDIAN (2102a) or KARDIA PHONE (2102b) may be more convenient for periodic use at home or during travel. Moreover, a patient may alternately use different combinations of one or more available input sources to monitor cardiac and other physiologic activity. The system 2100 is configured to accept different combinations of signals from input sources 2102a-h and to determine estimated analyte levels based on the different combinations of signals.

In some implementations, the system 2100 includes a device translator 2104, a multi-input processing engine 2106, and an analyte estimation model 2108. Each of these components 2104, 2106, and 2108 may be implemented on a system of one or more computers in one or more locations. The device translator 2104 prepares signals generated by the various input sources 2102a-h for processing by the multi-input processing engine 2106. The device translator 2104 may classify a given signal as having originated from a particular source 2102 and may classify the type of signal, e.g., as a single-lead ECG signal, a multi-lead ECG signal, an orientation or motion signal from an accelerometer or other orientation or motion sensing device, or a respiratory signal. In some implementations, input sources 2104 may be pre-registered with the system 2100 so the device translator 2104 can automatically identify the source and type of an input signal by comparing the signal, or metadata associated with the signal, to stored registration information for each of the devices. The device translator 2104 may also adapt signals from one or more sources for processing by the multi-input processing engine 2106. Signal adaptation can include normalizing signals with respect to each other and synchronizing signals in the time domain. In some implementations, further signal adaptation is performed by the multi-input processing engine 2106.

The multi-input processing ("MIP") engine 2106 is configured to process signals from one or more input sources 2102. The MIP engine 2106 may identify a requested task and determine how to process the signals based on the identified task. For example, a first set of ECG waveform features may be extracted if the task is to estimate a level of Magnesium in a patient's bloodstream, but a different second set of ECG waveform features may be extracted if the task is to estimate a level of Potassium in the patient's bloodstream. The MIP engine 2106 may also determine a manner of processing the input signals based on which signals are available for a given task. For example, if concurrently recorded ECG signals, motion-orientation signals, and respiratory signals are all available, the MIP engine 2106 may align and time stamp (e.g., synchronize) the signals, combine like signals (e.g., determine a signal-averaged beat from multiple ECG signals from different sources), and extract feature values from the signals.

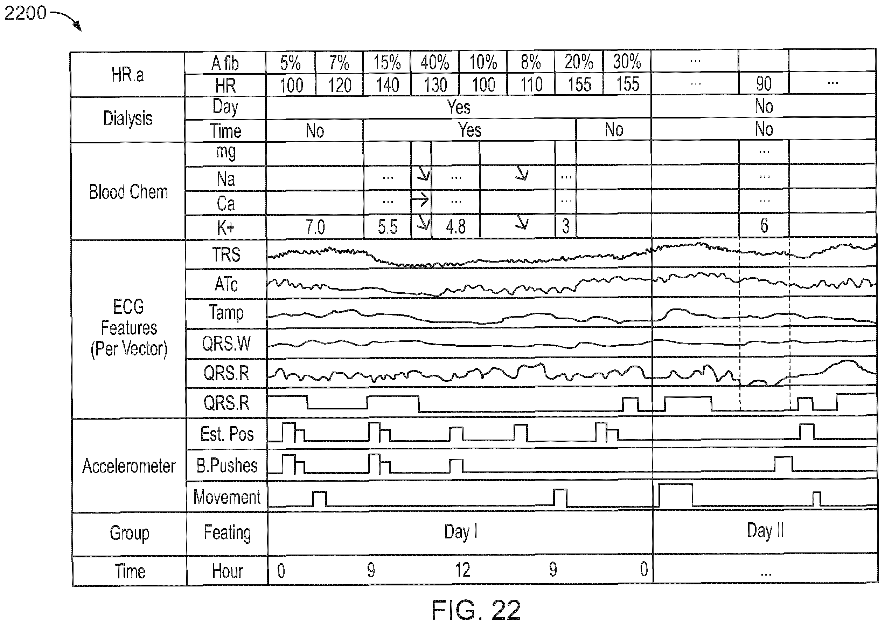

The analyte estimation model 2108 is configured to generate estimations of analyte levels for one or more analytes based on data provided by MIP engine 2106. The model 2108 may be trained on labeled data that associates known analyte levels (e.g., measured by a blood test) with features of one or more types of input data, including input data selected from the group comprising ECG data, heartrate, body position/pose, and respiratory data. Figure 22, for example, shows a time-aligned plot 2200 of features from multiple types of input data that the model 2108 may process to generate estimated analyte levels for a patient. Among the features or parameters that the model 2108 may process are heartrate, level of atrial fibrillation, time of day (e.g., morning, afternoon, evening), measured blood levels at particular times, ECG waveform features, and accelerometer data. The model 2108 may take various forms, including one or more of a neural network, a classifier, a linear regression model, and a hidden Markov model. The models may be trained, for example, using deep learning or reinforced learning techniques.

*Example Implementation:*

Elsewhere, in a small cohort of dialysis patients, it was demonstrated that the signal processed 12-lead ECG can detect subtle T-wave changes that in turn can be used to determine blood potassium concentrations reliably. *See* Dillon JJ, DeSimone CV, Sapir Y, Somers VK, Dugan JL, Bruce CJ, Ackerman MJ, Asirvatham SJ, Striemer BL, Bukartyk J: Noninvasive potassium determination using a mathematically processed ECG: Proof of concept for a novel "blood-less, blood test". Journal of electrocardiology 48, 12-18 (2015). In order to facilitate clinical applicability, a less cumbersome non-invasive approach may be preferred. To that end, a goal of this study was to refine our processing methodology to reduce detection requirements to a single channel (to enable mobility and home use), and to demonstrate not only a correlation between the processed ECG and potassium, but to use the ECG to prospectively calculate potassium values reliably. Potassium value extraction utilizing a single lead may permit use in wearable, wireless ECG patches, and possibly in implantable loop recorders and cardiac implantable electronic devices (pacemakers and defibrillators). In order to test the hypothesis that the properly processed ECG could be used to determine serum potassium from a single lead, and to do this reliably both with and without an initial "seeding" blood test to train the algorithm, we performed a prospective trial in a cohort of dialysis patients.

*Methods.* Inpatients and outpatients aged 18 years and older undergoing clinically indicated hemodialysis at Mayo Clinic in Rochester, MN, were prospectively enrolled under IRB approved protocols after providing written, informed consent. In all patients, 12-lead ECG data were acquired using electrodes in standard clinical positions recorded with a Siesta 802 system (Compumedics, Charlotte, NC) starting immediately before the onset of dialysis and continuing until its termination. Signal acquisition was performed at a rate of 1024 samples per seconds. Data were analyzed using the Matlab environment (MathWorks Natick, Massachusetts, USA).

*Patient Groups.* Patients in the algorithm development group, Group 1, consisted of 26 individuals who underwent three dialysis sessions as part of the study. At each dialysis session blood was drawn for analysis at three time points: pre-dialysis; at the mid-time point of dialysis after temporarily clamping the heparin line (if in use), stopping dialysate flow and decreasing the blood flow rate to 100 mL/min for at least 15 seconds; and post-dialysis after stopping dialysate flow and decreasing the blood flow rate to 100 ml/min for at least 15 seconds. This group was used to develop the algorithm tested in this report. While the algorithm applied concepts developed elsewhere (*see* Dillon JJ, DeSimone CV, Sapir Y, Somers VK, Dugan JL, Bruce CJ, Ackerman MJ, Asirvatham SJ, Striemer BL, Bukartyk J: Noninvasive potassium determination using a mathematically processed ECG: Proof of concept for a novel "blood-less, blood test". Journal of electrocardiology 48, 12-18 (2015)), the filtering and processing were included improvements to better account for ambient electrical noise and intermittent poor signal quality, and to permit single lead recording, as detailed below.

The validation group was composed of two sub-groups, Group 2A and Group 2B. Group 2A consisted of 8 patients, each of whom underwent three recorded dialysis sessions with two blood tests, one before and one immediately following dialysis. Group 2B consisted of 11 patients who had been previously studied so that full dialysis and digitized ECG data were available. *See* Dillon JJ, DeSimone CV, Sapir Y, Somers VK, Dugan JL, Bruce CJ, Ackerman MJ, Asirvatham SJ, Striemer BL, Bukartyk J: Noninvasive potassium determination using a mathematically processed ECG: Proof of concept for a novel "blood-less, blood test". Journal of electrocardiology 48, 12-18 (2015). None of the data from the Group 2 patients was used to create the potassium predication algorithms described in this manuscript.

*Analysis.* Results are represented as means ± standard deviation unless otherwise noted. To compare prediction performance of different prediction models, absolute errors were calculated between observed and predicted measurements and summarized.

*Personalized Analysis Strategy.* For each patient in Group 1, a single dialysis session was used to identify each patient's potassium "dose-response curve" that defined the relationship between the processed ECG parameter and the measured potassium value for each individual. Of the three dialysis sessions, the first was used to seed the algorithm by defining the processed ECG parameter-potassium relationship. The ECG data from the second and third dialysis session were then used to calculate potassium values, and the blood tests used to calculate the error in the calculated potassium. Thus, while Group 1 was defined as the algorithm development group, for the purposes of the *personalized* analysis the first session was used for personalization, and the next two sessions used to test the results of the personalized strategy.

*Global Analysis Strategy.* The global analysis strategy assumed that the relationship between the signal processed ECG and blood potassium is universal, or at least stable for humans (species specific), as opposed to specific for each individual, and that potassium determination could therefore be performed without seeding the algorithm using blood tests from each individual (a completely blood-free "bloodless blood test"). In order to perform the *global* analysis, we combined all of the data from the first dialysis session of all patients in Group 1 into a composite group. This was used to create a *global* model of the relationship between the signal processed ECG and serum potassium. This global model was tested in two ways. First, we assessed the ability of this model to determine the potassium during the second and third sessions for the patients in Group 1. In other words, we first tested the model's ability to determine potassium in subsequent dialysis sessions using the same cohort that developed the model. Next, we tested the global model by applying it to the patients in Group 2 (validation group), none of whom contributed data to model creation.

*ECG Signal Processing and Analysis*

*Electrode Selection and Segmentation.* ECG data from all patients were processed using a multistage signal-averaging algorithm. In this work, we sought to analyze electrodes in similar precordial positions between sessions, to mimic anticipated deployment using prolonged monitoring via a single channel patch or a subcutaneous device. To accomplish this, for each session, only data from the single lateral precordial lead (i.e. one of V3-V6) with the greatest amplitude T-wave was used to predict potassium. Signal amplitude between sessions was normalized to the square root of the T-wave amplitude, although the signal amplitude could be normalized by other features as well (e.g., T-wave amplitude, T-wave area). We used this as our first generation approach to minimize electrode placement wander, as more advanced strategies are under development. Additionally, in this early demonstration project, we only included patients with a positive uniphasic T-wave in the lead under analysis. The ECG data were divided into 72 second segments, to allow overlap of one minute intervals. The processing algorithms were then applied to each of the segments. This resulted in every one-minute segment having a processed, filtered, averaged representative ECG complex. This processed ECG complex was used for morphologic feature extraction.

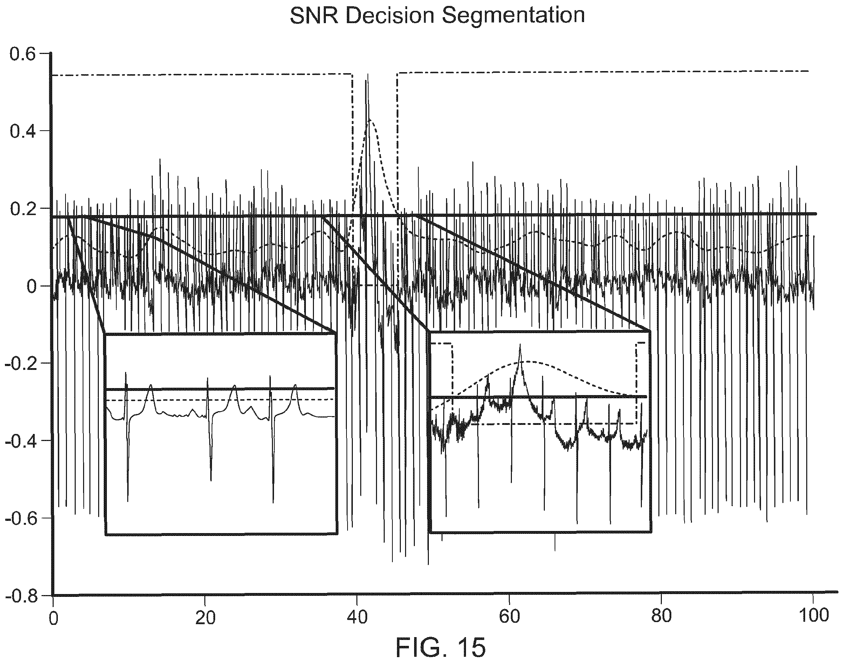

*Signal Processing and Averaging, and "Big Data" Strategy.* In each 72 second high resolution single-lead ECG segment, there were typically between 50-200 beats (depending on ectopy/filtering, described below) that were averaged and processed to a single representative complex. This resulted in a large ratio between the input and the output of the data processing algorithm, creating significant data redundancy. The robust data redundancy permitted ECG signal cleaning utilizing an improved strategy - an artifact detector - that rejected suboptimal data to permit high fidelity analysis from a single electrode, as opposed to filtering the data and introducing possible distortions. The artifact detector identified changes in the baseline using linear and non-linear filtering of the ECG at low frequencies. It automatically scored the signal and applied an adaptive threshold chosen using the entire segment's mean and median score. Any section of signal having a score that exceeded the threshold was defined as contaminated with artifact and discarded (Figure 15). The second step of the algorithm involved Detection of QRS Complexes and a correlation filter that eliminated ectopic and aberrant complexes. *See* Zidelmal, Z., Amirou, A., Adnane, M. & Belouchrani, A. QRS detection based on wavelet coefficients. Comput. Methods Prog. Biomed. 107, 490-496, doi:10.1016/j.cmpb.2011.12.004 (2012); Seena, V. & Yomas, J. in Devices, Circuits and Systems (ICDCS), 2014 2nd International Conference on. 1-6. In the third step of the algorithm, all complexes were aligned using a fiducial point in the QRS, and complexes that varied from the median pattern were removed. In the final step, complexes were signal averaged to remove noise and smooth the waveform morphology.

*ECG Feature Extraction.* The averaged, processed ECG complex was used for feature extraction for analysis. T-wave peak and T-wave end points were selected in an automated manner as described elsewhere. *See* Dillon JJ, DeSimone CV, Sapir Y, Somers VK, Dugan JL, Bruce CJ, Ackerman MJ, Asirvatham SJ, Striemer BL, Bukartyk J: Noninvasive potassium determination using a mathematically processed ECG: Proof of concept for a novel "blood-less, blood test". Journal of electrocardiology 48, 12-18 (2015). The algorithm then automatically selected a representative section of the descending T-wave to estimate its slope (T-right slope) using the mean derivative approach. *See* Hamming, R. Numerical methods for scientists and engineers. (Courier Corporation, 2012); Zhang, Q., Manriquez, A. I., Medigue, C., Papelier, Y. & Sorine, M. An algorithm for robust and efficient location of T-wave ends in electrocardiograms. Biomedical Engineering, IEEE Transactions on 53, 2544-2552 (2006); Helfenbein ED, Ackerman MJ, Rautaharju PM, Zhou SH, Gregg RE, Lindauer JM, Miller D, Wang JJ, Kresge SS, Babaeizadeh S: An algorithm for QT interval monitoring in neonatal intensive care units. Journal of electrocardiology 40, S103-S110 (2007). T-wave amplitude (T Amp) was measured as the difference in millivolts between the T peak and T end. After deriving these values of the T-right slope and T-amp, a Kalman filter (*see* Kalman, R. E. A new approach to linear filtering and prediction problems. Journal of Fluids Engineering 82, 35-45 (1960)) was used to reduce noise, taking advantage of the fact that the rate of serum potassium change over 72 seconds is limited, and abrupt segmental changes represent a segmental anomaly as opposed to a true potassium change.

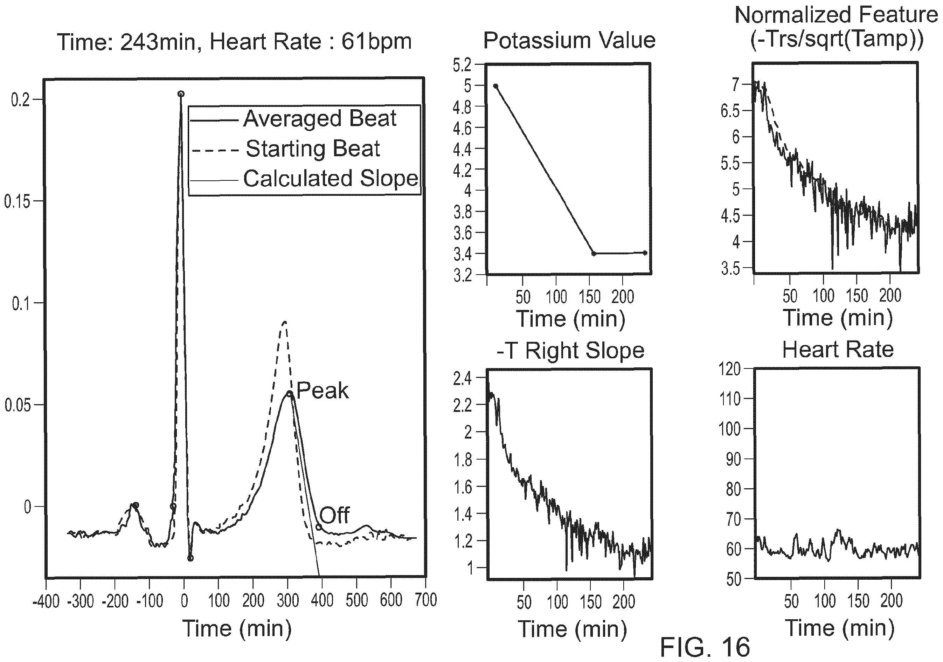

*Potassium and Feature Extraction During Dialysis.* To validate the correlation between the features selected and potassium values, we built a tool for temporal progression analysis. This tool permitted "fast-forward" ECG analysis using the methods described elsewhere. The tool demonstrates the progress of the automated ECG analysis on the dialysis time line and representative corresponding potassium blood tests in a time-lapse manner. An example of tool usage to analyze feature extraction during dialysis run is shown in Figure 16.

*Creation of Prediction Models.* With the personalized strategy, potassium values from the first dialysis session and $\frac{T-right\ slope}{\sqrt{T\ Amp}}$ were used to build a linear least square estimator for each patient. The second and third ECG recordings were extracted, processed and plugged into the estimator for predication purposes.

In the global prediction approach, we combined all group 1 patients' first visit data to create a "global estimator." To combine the ECGs from all patients, we normalized the T-right slope with the Square root of the T-Amp.

*Results.* We recorded ECG data during 129 dialysis sessions in 51 patients, with a mean of 2.5 sessions per patient. Patients had a mean age of 58+/-16 years and 66% were men. The mean left ventricular ejection fraction (LVEF) was 59 $\pm$7, and 9% of analyzed patients had a history of myocardial infraction. In those patients excluded from the analysis due to unusable ECG, LVEF was 46$\pm$16% and 67% of patients had a history of myocardial infarction. Biphasic or inverted T-waves precluded analysis of any data in six patients (11% of the study population) and precluded analysis of a single visit from one patient.

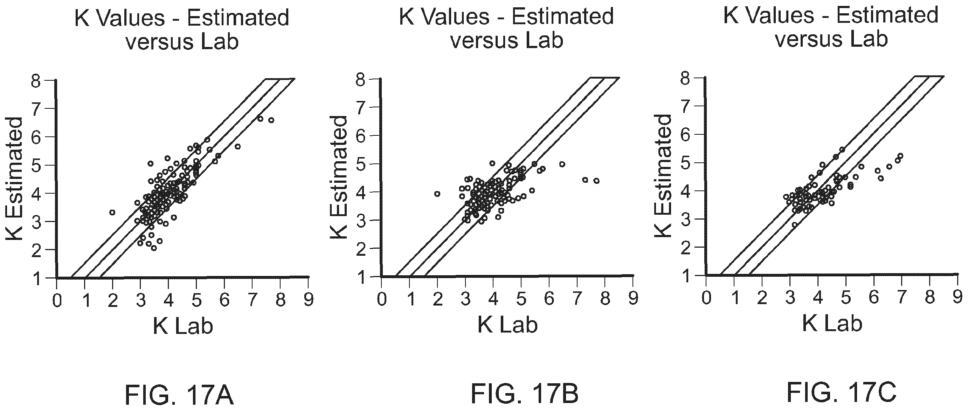

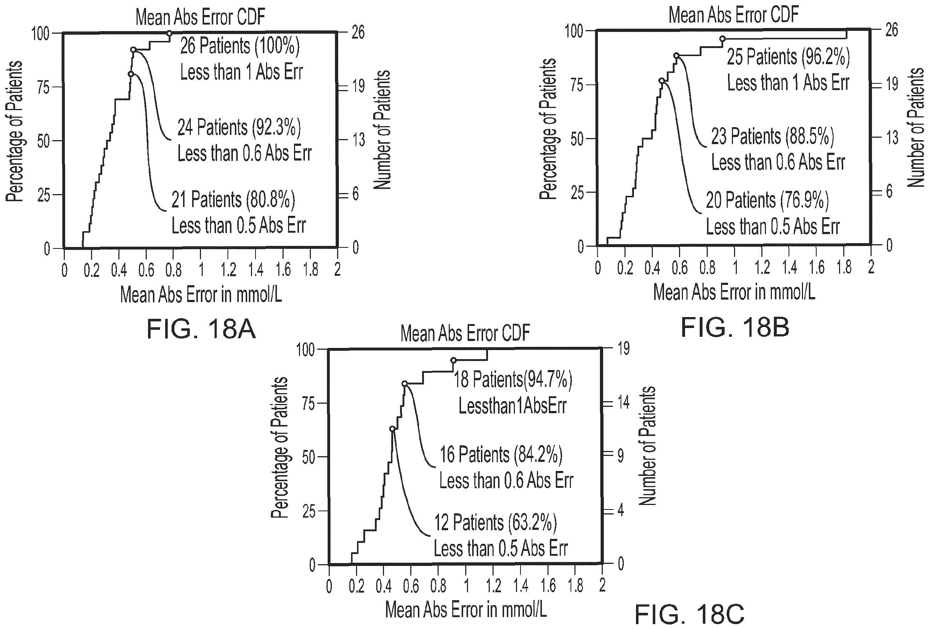

*Personalized Analysis.* The personalized estimator was tested on 26 patients who had all three visits with three blood tests during each visit, except for three patients in whom one blood test or an ECG at the time of phlebotomy was not available. The measured blood potassium value was 3.9 $\pm$0.8 mmol/L. The mean absolute error across two subsequent visits (6 blood tests total per patient) was 0.36 $\pm$ 0.34 mmol/L. The median absolute error was 0.26 mmol/L and the averaged percentage error was 10% of the serum potassium blood test result (Figures 17 and 18).

*Global Analysis (no seeding blood test).* The global estimator was tested in two populations. The first population tested was Group 1 (the development group) in which the global estimator was used to calculate the potassium in the second and third dialysis sessions. The absolute error value in two different visits (6 blood tests total) was 0.44 $\pm$ 0.47 mmol/L, the median absolute error was 0.33 mmol/L and the averaged percentage error was 11% of the serum potassium blood test result. Using the square root of the T-wave to normalize the t right slope improved the results of the global analysis.

When the global analysis was applied in Group 2 (validation population, none of whose data contributed to model creation), the measured blood potassium value was 4.2 $\pm$ 0.95 mmol/L. The absolute error value in 6 blood tests for group 2A and 3 blood tests for group 2B was 0.5 $\pm$ 0.42 mmol/L; median error was 0. 41 mmol/L, and averaged percentage error was 12% of the serum potassium blood test result.

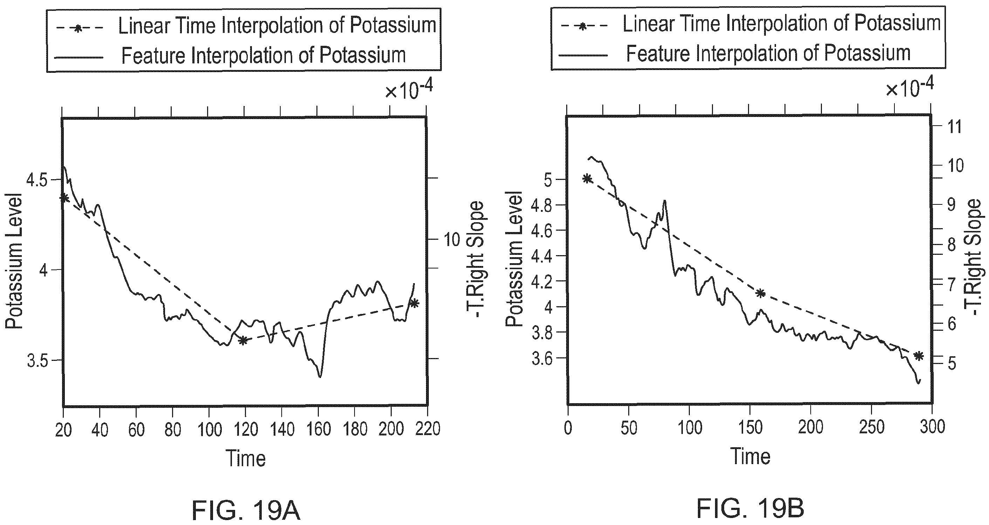

The temporal progression tool confirmed the utility of using the T-wave right slope as a parametric feature to calculate potassium. Additionally, it confirmed that the temporal change in calculated potassium paralleled changes in the blood tests, and suggested a more accurate means of assessing potassium values during dialysis (figure 19).

*Discussion.* In patients with cardiovascular and/or renal disease, hyperkalemia is frequent, life-threatening, and usually asymptomatic. *See* Gennari, F. J. Hypokalemia. New England Journal of Medicine 339, 451-458, doi:doi:10.1056/NEJM199808133390707 (1998); Weiner, I. D. & Wingo, C. S. Hyperkalemia: a potential silent killer. JOURNAL-AMERICAN SOCIETY OF NEPHROLOGY 9, 1535-1543 (1998). The emergence of safe and effective medications that lower potassium underscores the importance of detection of hyperkalemia. *See* Ingelfinger, J. R. A New Era for the Treatment of Hyperkalemia? New England Journal of Medicine 372, 275-277, doi:doi:10.1056i-NEJMe1414112 (2015); Ash, S. R., Singh, B., Lavin, P. T, Stavros, F. & Rasmussen, H. S. A phase 2 study on the treatment of hyperkalemia in patients with chronic kidney disease suggests that the selective potassium trap, ZS-9, is safe and efficient. Kidney Int, doi:10.1038/ki.2014.382 (2015); Packham DK, Rasmussen HS, Lavin PT, El-Shahawy MA, Roger SD, Block G, Qunibi W, Pergola P, Singh B: Sodium Zirconium Cyclosilicate in Hyperkalemia. New England Journal of Medicine 372, 222-231, doi:doi:10.1056/NEJMoa1411487 (2015); Kosiborod M, Rasmussen HS, Lavin P, Qunibi WY, Spinowitz B, Packham D, Roger SD, Yang A, Lerma E, Singh B. Effect of sodium zirconium cyclosilicate on potassium lowering for 28 days among outpatients with hyperkalemia: The harmonize randomized clinical trial. JAMA 312, 2223-2233, doi:10.1001/jama.2014.15688 (2014). The ability to remotely, unobtrusively, frequently, and non-invasively assess potassium through a single channel signal processed ECG would permit currently available wireless ECG patches, implanted monitors, and cardiac devices to infer measurements of potassium, and would address a critical need, impacting a large population. In this study of patients undergoing hemodialysis, the signal processed ECG was able to determine potassium values with a mean error of $0.36 \pm 0.34$ mmol/L when using a personalized strategy that required a "seeding blood test," providing a clinically meaningful value via individualized medicine. Importantly, even without personalization and in the absence of any blood draws, clinically useful estimates of potassium were obtained, providing potassium values with a mean error of $0.5 \pm 0.42$ mmol/L, useful for alerts and trending. These findings, using a single lead of high resolution ECG data, suggest that this approach may be suitable to remotely monitor potassium in dialysis patients. This is a high risk population for hyperkalemia and sudden death, often in the 12 hours before a dialysis session, suggesting a hyperkalemic mechanism. [1]

Several algorithmic strategies were applied to achieve a high level of precision. One was application of an "artifact detector" concept, in which the availability of redundant data permitted use of an automated artifact detector that discarded poor quality data rather than attempt to filter or clean it. This fundamental strategy may be applicable to the analysis of a large number of physiologic signals for which mild or moderate latency is tolerable. Given that potassium values are generally not available clinically in the absence of blood tests, even once or twice daily assessments may represent a significant advance, particularly in high risk patients recently dismissed from hospital. Delays in determination of a potassium value of minutes or hours can be acceptable clinically. Broadly, a major challenge in remote patient monitoring is the issue of artifact and noise, commonly present when non-obtrusive, well-tolerated sensors are used to acquire often-noisy signals in ambulatory individuals. Such challenge can be substantially overcome by leveraging data redundancy. A second strategy was the use of the Kalman filter - which has historically been used to distinguish returning radar signals caused by flocks of birds from those of tracked airplanes by recognizing constraints in the abruptness of change of trajectory and velocity of which an airplane is capable. In a similar manner, we recognize that a marked change in potassium over a time frame of a few minutes, particularly if not a consistent change, represents measurement error, permitting correction and increased accuracy.

In this study we used advanced algorithms to further validate the use of easily obtained electrocardiographic repolarization to predict potassium in both personalized and global predication models in hemodialysis patients. We focused on single lead recordings to allow practical implementation and employed two T-wave features that were found to correlate with potassium levels. *See* Dillon JJ, DeSimone CV, Sapir Y, Somers VK, Dugan JL, Bruce CJ, Ackerman MJ, Asirvatham SJ, Striemer BL, Bukartyk J: Noninvasive potassium determination using a mathematically processed ECG: Proof of concept for a novel "blood-less, blood test". Journal of electrocardiology 48, 12-18 (2015). These were used to develop both the personalized and global predictor models. The use of the descending T-wave in lateral precordial leads mechanistically corroborates the relationship between potassium and repolarization. Extracellular potassium can differentially affects the action potential repolarization in mid-myocardial as compared to endocardial and epicardial myocytes, reflected predominantly on the surface ECG as the T-wave right slope. *See* Yan, G.-X. & Antzelevitch, C. Cellular basis for the normal T wave and the electrocardiographic manifestations of the long-QT syndrome. Circulation 98, 1928-1936 (1998). Changes in extra-cellular potassium concentrations affect the transmembrane voltage gradient of each myocyte, in aggregate summarized as the surface T-wave. The function of potassium channels is essential to life, and their genetic sequence highly conserved on an evolutionary scale, with similar sequences in species ranging from bacteria to humans. *See* Choe, S. Potassium channel structures. Nature Reviews Neuroscience 3, 115-121 (2002). Thus, transmembrane channels are ideal "micro-sensors" of potassium levels, and global analysis is feasible, supporting the concept that we are detecting the sum of potassium changes at the cellular level, accounting for our unique fidelity in detecting subtle changes.

Elsewhere, the correlation between the T right slope and T amplitude and potassium has been described. In this work, we use the correlation in a personalized and global predictive manner. Corsi *et al.* found a similar relationship between the T-wave and potassium levels, corroborating our findings. *See* Corsi C, DeBie J, Napolitano C, Priori S, Mortara D, Severi S: Validation of a novel method for non-invasive blood potassium quantification from the ECG. Computing in Cardiology (CinC), 2012. 105-108. However, they created multidimensional Eigen-leads using principal component analysis, which required a 12-lead ECG. While such a strategy further supports the concept, it may be impractical for ambulatory patient home use. We specifically developed tools that can permit adaptation to ambulatory patients. Additionally, we also used a different normalization method, by using square root of the T amp instead of the T amp itself, and therefore preserved some of the information yielded by the T-wave amplitude, while at the same time minimizing the "between patients" and "between visits" ECG variability. Finally, our prediction was based on only 5 minutes of ECG instead of 15, which may be more practical for remote monitoring applications.

The temporal progression tool we developed created an animation of the processed ECG-calculated potassium in a "time lapse" manner, permitting assessment of potassium change during dialysis to facilitate assessment of the impact of algorithm changes during development (Figure 16). Additionally, using the tool it is apparent that even if there is an error in absolute potassium value, the trend of the calculated potassium is very similar to the blood-test potassium trend, and indeed it may represent a more accurate assessment than the presumed linearity plotted between blood draw potassium values (Figure 19). Furthermore, such information may prove helpful both for determining whether an additional dialysis session may be helpful, to identify interventions needed to lower (or raise) potassium, and also for guiding the duration and intensity of dialysis itself.

In this initial study, patients with biphasic, bimodal or inverted T-waves were excluded, resulting in the exclusion of 6 patients (11.7% of enrolled subjects) from analysis, 66% of whom had a history of myocardial infarction. In one additional patient, a single dialysis session was excluded due to the presence of T-wave abnormalities at that visit alone. Patients with active ischemia and acute infarction likely will not be good candidates for this methodology; however, they are not typically treated via remote monitoring, and access to blood tests is not generally a challenge.

A number of factors other than potassium levels affect the ECG These include, but are not limited to, variations in lead position (*see* Kania M, Rix H, Fereniec M, Zavala-Fernandez H, Janusek D, Mroczka T, Stix G, Maniewski R: The effect of precordial lead displacement on ECG morphology. Medical & biological engineering & computing 52, 109-119 (2014*)),* alteration in body position, changes in weight and volume status (*see* Nguyên UC, Potse M, Regoli F, Caputo ML, Conte G, Murzilli R, Muzzarelli S, Moccetti T, Caiani EG, Prinzen FW: An in-silico analysis of the effect of heart position and orientation on the ECG morphology and vectorcardiogram parameters in patients with heart failure and intraventricular conduction defects. Journal of Electroccardiology (2015)), alterations in heart rate and rhythm, and the development of cardiac ischemia, as well as other electrolytes that change during dialysis. Some of these, most notably body and lead position variability and fluid volume changes, likely account for some of the estimation error. Several potential sources of error can be eliminated using both ECG template analysis and/or a sensor-based accelerometer to record body position. Despite the multiple potential sources of error, in this stable dialysis population, the mean ECG-derived potassium error was only $0.5 \pm 0.42$ mmol/L, or 12% on average. Lastly, we focused initially on potassium because of its clinical importance, and its known relationship with the ECG. However, certain ECG changes may be attributable to the concentrations of other analyte levels in the patient's bloodstream, and those ECG changes can thus be analyzed to provide estimates of blood concentrations of elements beyond potassium.

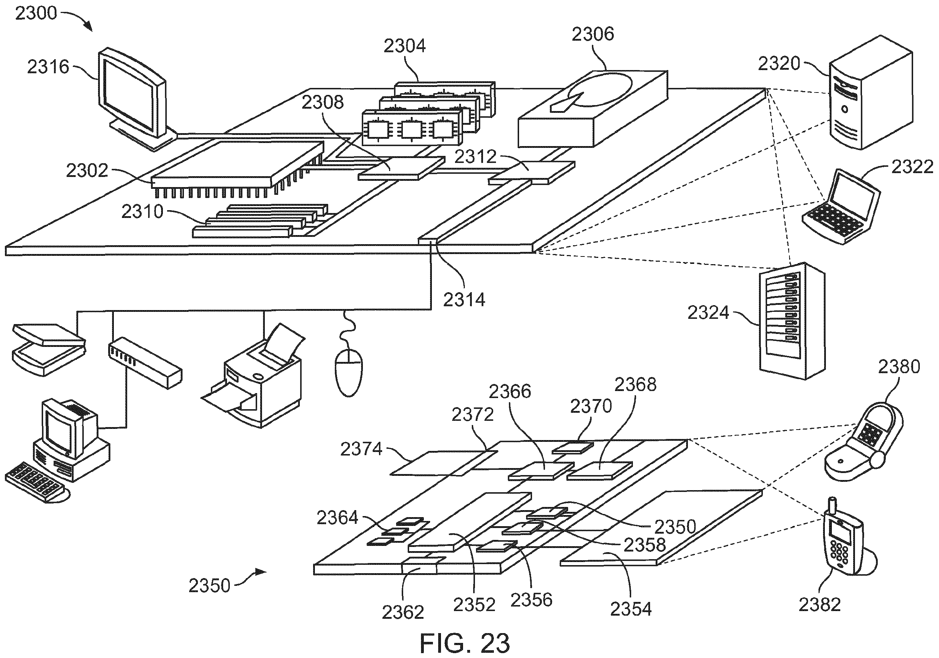

FIG. 23 is a block diagram of computing devices 2300, 2350 that may be used to implement the systems and methods described in this document, as either a client or as a server or plurality of servers. Computing device 2300 is intended to represent various forms of digital computers, such as laptops, desktops, workstations, personal digital assistants, servers, blade servers, mainframes, and other appropriate computers. Computing device 2350 is intended to represent various forms of mobile devices, such as personal digital assistants, cellular telephones, smartphones, and other similar computing devices. Additionally computing device 2300 or 2350 can include Universal Serial Bus (USB) flash drives. The USB flash drives may store operating systems and other applications. The USB flash drives can include input/output components, such as a wireless transmitter or USB connector that may be inserted into a USB port of another computing device. The components shown here, their connections and relationships, and their functions, are meant to be exemplary only, and are not meant to limit implementations described and/or claimed in this document.

Computing device 2300 includes a processor 2302, memory 2304, a storage device 2306, a high-speed interface 2308 connecting to memory 2304 and high-speed expansion ports 2310, and a low speed interface 2312 connecting to low speed bus 2314 and storage device 2306. Each of the components 2302, 2304, 2306, 2308, 2310, and 2312, are interconnected using various busses, and may be mounted on a common motherboard or in other manners as appropriate. The processor 2302 can process instructions for execution within the computing device 2300, including instructions stored in the memory 2304 or on the storage device 2306 to display graphical information for a GUI on an external input/output device, such as display 2316 coupled to high speed interface 2308. In other implementations, multiple processors and/or multiple buses may be used, as appropriate, along with multiple memories and types of memory. Also, multiple computing devices 2300 may be connected, with each device providing portions of the necessary operations (e.g., as a server bank, a group of blade servers, or a multi-processor system).

The memory 2304 stores information within the computing device 2300. In one implementation, the memory 2304 is a volatile memory unit or units. In another implementation, the memory 2304 is a non-volatile memory unit or units. The memory 2304 may also be another form of computer-readable medium, such as a magnetic or optical disk.

The storage device 2306 is capable of providing mass storage for the computing device 2300. In one implementation, the storage device 2306 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 2304, the storage device 2306, or memory on processor 2302.

The high speed controller 2308 manages bandwidth-intensive operations for the computing device 2300, while the low speed controller 2312 manages lower bandwidth-intensive operations. Such allocation of functions is exemplary only. In one implementation, the high-speed controller 2308 is coupled to memory 2304, display 2316 (e.g., through a graphics processor or accelerator), and to high-speed expansion ports 2310, which may accept various expansion cards (not shown). In the implementation, low-speed controller 2312 is coupled to storage device 2306 and low-speed expansion port 2314. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 2300 may be implemented in a number of different forms, as shown in the figure. For example, it may be implemented as a standard server 2320, or multiple times in a group of such servers. It may also be implemented as part of a rack server system 2324. In addition, it may be implemented in a personal computer such as a laptop computer 2322. Alternatively, components from computing device 2300 may be combined with other components in a mobile device (not shown), such as device 2350. Each of such devices may contain one or more of computing device 2300, 2350, and an entire system may be made up of multiple computing devices 2300, 2350 communicating with each other.

Computing device 2350 includes a processor 2352, memory 2364, an input/output device such as a display 2354, a communication interface 2366, and a transceiver 2368, among other components. The device 2350 may also be provided with a storage device, such as a microdrive or other device, to provide additional storage. Each of the components 2350, 2352, 2364, 2354, 2366, and 2368, are interconnected using various buses, and several of the components may be mounted on a common motherboard or in other manners as appropriate.

The processor 2352 can execute instructions within the computing device 2350, including instructions stored in the memory 2364. The processor may be implemented as a chipset of chips that include separate and multiple analog and digital processors. Additionally, the processor may be implemented using any of a number of architectures. For example, the processor 2352 may be a CISC (Complex Instruction Set Computers) processor, a RISC (Reduced Instruction Set Computer) processor, or a MISC (Minimal Instruction Set Computer) processor. The processor may provide, for example, for coordination of the other components of the device 2350, such as control of user interfaces, applications run by device 2350, and wireless communication by device 2350.

Processor 2352 may communicate with a user through control interface 2358 and display interface 2356 coupled to a display 2354. The display 2354 may be, for example, a TFT (Thin-Film-Transistor Liquid Crystal Display) display or an OLED (Organic Light Emitting Diode) display, or other appropriate display technology. The display interface 2356 may comprise appropriate circuitry for driving the display 2354 to present graphical and other information to a user. The control interface 2358 may receive commands from a user and convert them for submission to the processor 2352. In addition, an external interface 2362 may be provide in communication with processor 2352, so as to enable near area communication of device 2350 with other devices. External interface 2362 may provide, for example, for wired communication in some implementations, or for wireless communication in other implementations, and multiple interfaces may also be used.

The memory 2364 stores information within the computing device 2350. The memory 2364 can be implemented as one or more of a computer-readable medium or media, a volatile memory unit or units, or a non-volatile memory unit or units. Expansion memory 2374 may also be provided and connected to device 2350 through expansion interface 2372, which may include, for example, a SIMM (Single In Line Memory Module) card interface. Such expansion memory 2374 may provide extra storage space for device 2350, or may also store applications or other information for device 2350. Specifically, expansion memory 2374 may include instructions to carry out or supplement the processes described above, and may include secure information also. Thus, for example, expansion memory 2374 may be provide as a security module for device 2350, and may be programmed with instructions that permit secure use of device 2350. In addition, secure applications may be provided via the SIMM cards, along with additional information, such as placing identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 2364, expansion memory 2374, or memory on processor 2352 that may be received, for example, over transceiver 2368 or external interface 2362.

Device 2350 may communicate wirelessly through communication interface 2366, which may include digital signal processing circuitry where necessary. Communication interface 2366 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 2368. In addition, short-range communication may occur, such as using a Bluetooth, WiFi, or other such transceiver (not shown). In addition, GPS (Global Positioning System) receiver module 2370 may provide additional navigation- and location-related wireless data to device 2350, which may be used as appropriate by applications running on device 2350.

Device 2350 may also communicate audibly using audio codec 2360, which may receive spoken information from a user and convert it to usable digital information. Audio codec 2360 may likewise generate audible sound for a user, such as through a speaker, e.g., in a handset of device 2350. Such sound may include sound from voice telephone calls, may include recorded sound (e.g., voice messages, music files, etc.) and may also include sound generated by applications operating on device 2350.

The computing device 2350 may be implemented in a number of different forms, as shown in the figure. For example, it may be implemented as a cellular telephone 2380. It may also be implemented as part of a smartphone 2382, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), peer-to-peer networks (having ad-hoc or static members), grid computing infrastructures, and the Internet.

The computing system can include clients and servers. A client and server are generally remote from each other and typically interact through a communication network. The relationship of client and server arises by virtue of computer programs running on the respective computers and having a client-server relationship to each other.

Although a few implementations have been described in detail above, other modifications are possible. Moreover, other mechanisms quantifying potassium based on ECG data may be used. In addition, the logic flows depicted in the figures do not require the particular order shown, or sequential order, to achieve desirable results. Other steps may be provided, or steps may be eliminated, from the described flows, and other components may be added to, or removed from, the described systems. Accordingly, other implementations are within the scope of the following claims.

EMBODIMENTS

1. A computer-implemented method for assessing a level of an analyte within a human, the method comprising:

obtaining data indicating electrocardiogram results from the human;

identifying a plurality of beats represented in the electrocardiogram results;

determining, for each beat in the plurality of beats represented in the

electrocardiogram results, a value for a first feature of the beat;

performing statistical analysis on the values for the first feature of the plurality of beats;

generating an indication of the level of the analyte within the human based on a result of the statistical analysis performed on the values for the first feature of the plurality of beats; and

providing the indication of the level of the analyte within the human.

2. The computer-implemented method of embodiment 1, wherein the analyte is potassium.

3. The computer-implemented method of embodiment 1, wherein the analyte is selected from a group consisting of potassium, magnesium, phosphorous, calcium, bicarbonate, hydrogen ion, and glucose.

4. The computer-implemented method of embodiment 1, wherein determining, for each beat in the plurality of beats represented in the electrocardiogram results, the value for the first feature of the beat comprises calculating, for each beat in the plurality of beats, a slope of at least a portion of a T-wave in the beat between the peak of the T-wave and the end of the T- wave.

5. The computer-implemented method of embodiment 1, wherein determining, for each beat in the plurality of beats represented in the electrocardiogram results, the value for the first feature of the beat comprises calculating, for each beat in the plurality of beats, a magnitude of the peak of a T-wave in the beat.

6. The computer-implemented method of embodiment 1, further comprising: determining, for each beat in the plurality of beats represented in the electrocardiogram results, a value for a second feature of the beat other than the first feature; and

performing statistical analysis on the values for the second feature of the plurality of beats;

wherein the indication of the level of the analyte within the human is generated further based on a result of the statistical analysis performed on the values for the second feature of the plurality of beats.

7. The computer-implemented method of embodiment 1, wherein the data indicating the electrocardiogram results from the human is data recorded from only a single lead of an electrocardiogram device.

8. The computer-implemented method of embodiment 1, wherein the data indicating the electrocardiogram results from the human is data recorded from a portable electrocardiogram device having less than 12 leads.

9. The computer-implemented method of embodiment 1, further comprising:

analyzing the data indicating the electrocardiogram results from the human to identify segments of the electrocardiogram results having a signal quality score that does not meet a threshold signal quality score; and

discarding the segments of the electrocardiogram results having the signal quality score that does not meet the threshold signal quality score.

10. The computer-implemented method of embodiment 9, wherein identifying the plurality of beats represented in the electrocardiogram results comprises identifying beats from segments of the electrocardiogram results other than the discarded segments having the signal quality score that does not meet the threshold signal quality score.

11. The computer-implemented method of embodiment 1, wherein performing statistical analysis on the values for the first feature of the plurality of beats comprises fitting a distribution of the values for the first feature of at least some of the plurality of beats to a probability distribution function.

12. The computer-implemented method of embodiment 11, wherein the probability distribution function is a normal probability distribution function, a gamma probability distribution function, or a Gaussian probability distribution function.

13. The computer-implemented method of embodiment 1, wherein generating the indication of the level of the analyte within the human comprises comparing the values for the first feature of at least a subset of the plurality of beats to a pre-defined template that maps values for the first feature to levels of the analyte within a human or that maps values derived at least in part from the first feature to levels of the analyte within a human.

14. The computer-implemented method of embodiment 13, wherein the pre-defined template is generated based on assessments of the level of the analyte within a population of humans other than the human from which the data indicating the electrocardiogram results was obtained.

15. The computer-implemented method of embodiment 13, wherein the pre-defined template is generated based on assessments of the level of the analyte within the human from which the data indicating the electrocardiogram results was obtained.

16. The computer-implemented method of embodiment 1, wherein generating the indication of the level of the analyte within the human comprises comparing the result of the statistical analysis performed on the values for the first feature of the plurality of beats to a pre-defined template that maps statistical results for historical values for the first feature to levels of the analyte within a human.

17. The computer-implemented method of embodiment 16, wherein the pre-defined template is generated based on assessments of the level of the analyte within a population of humans other than the human from which the data indicating the electrocardiogram results was obtained.

18. The computer-implemented method of embodiment 16, wherein the pre-defined template is generated based on assessments of the level of the analyte within the human from which the data indicating the electrocardiogram results was obtained.

19. The computer-implemented method of embodiment 16, wherein the method is performed at least in part on a mobile computing device of the human.

20. The computer-implemented method of embodiment 19, wherein providing the indication of the level of the analyte within the human comprises presenting the indication of the level of the analyte on an electronic display of the mobile computing device.

21. The computer-implemented method of embodiment 1, wherein identifying the plurality of beats comprises identifying QRS complexes that occur in at least a subset of the plurality of beats, wherein identifying the QRS complexes that occur in the at least the subset of the plurality of beats comprises identifying features that occur in the electrocardiogram results at a frequency indicated by the human's heart rate or by an estimate of the R-R interval in the electrocardiogram results.

22. The computer-implemented method of embodiment 1, further comprising generating an animation that shows changes in the electrocardiogram results and changes in the first feature over a period of time.

23. The computer-implemented method of embodiment 1, wherein determining the values for the first feature of the plurality of beats comprises determining initial values for the first feature of the plurality of beats and filtering the initial values.

24. The computer-implemented method of embodiment 23, wherein filtering the initial values for the first feature comprises applying a Kalman filter to the initial values.

25. The computer-implemented method of embodiment 1, further comprising modifying the data indicating the electrocardiogram results by demodulating a QRS envelope of the electrocardiogram results.

26. A computer-implemented method for assessing a level of an analyte within a human, the method comprising:

   generating electrocardiogram results from the human; and

   comparing the results to a signal template to obtain an indication of the level of the analyte in the human, wherein the signal template was generated based on assessments of the level of the analyte within a population of humans other than the human from which the

   electrocardiogram results were generated.

27. One or more non-transitory computer-readable devices having instructions stored thereon that, when executed by one or more processors, cause performance of operations comprising:

obtaining data indicating electrocardiogram results from the human;

identifying a plurality of beats represented in the electrocardiogram results;

determining, for each beat in the plurality of beats represented in the

electrocardiogram results, a value for a first feature of the beat;

performing statistical analysis on the values for the first feature of the plurality of beats;

generating an indication of the level of the analyte within the human based on a result of the statistical analysis performed on the values for the first feature of the plurality of beats; and

providing the indication of the level of the analyte within the human.

28. The one or more non-transitory computer-readable devices of embodiment 27, wherein the analyte is potassium.

29. The one or more non-transitory computer-readable devices of embodiment 27, wherein the analyte is selected from a group consisting of potassium, magnesium, phosphorous, calcium, bicarbonate, hydrogen ion, and glucose.

30. The one or more non-transitory computer-readable devices of embodiment 27, wherein determining, for each beat in the plurality of beats represented in the electrocardiogram results, the value for the first feature of the beat comprises calculating, for each beat in the plurality of beats, a slope of at least a portion of a T-wave in the beat between the peak of the T-wave and the end of the T-wave.

31. The one or more non-transitory computer-readable devices of embodiment 27, wherein determining, for each beat in the plurality of beats represented in the electrocardiogram results, the value for the first feature of the beat comprises calculating, for each beat in the plurality of beats, a magnitude of the peak of a T-wave in the beat.

32. The one or more non-transitory computer-readable devices of embodiment 27, wherein the operations further comprise:

determining, for each beat in the plurality of beats represented in the

electrocardiogram results, a value for a second feature of the beat other than the first feature; and

performing statistical analysis on the values for the second feature of the plurality of beats;

wherein the indication of the level of the analyte within the human is generated further based on a result of the statistical analysis performed on the values for the second feature of the plurality of beats.

33. The one or more non-transitory computer-readable devices of embodiment 27, wherein the data indicating the electrocardiogram results from the human is data recorded from only a single lead of an electrocardiogram device.

34. The one or more non-transitory computer-readable devices of embodiment 27, wherein the data indicating the electrocardiogram results from the human is data recorded from a portable electrocardiogram device having less than 12 leads.

35. The one or more non-transitory computer-readable devices of embodiment 27, wherein the operations further comprise:

analyzing the data indicating the electrocardiogram results from the human to identify segments of the electrocardiogram results having a signal quality score that does not meet a threshold signal quality score; and

discarding the segments of the electrocardiogram results having the signal quality score that does not meet the threshold signal quality score.

36. The one or more non-transitory computer-readable devices of embodiment 35, wherein identifying the plurality of beats represented in the electrocardiogram results comprises identifying beats from segments of the electrocardiogram results other than the discarded segments having the signal quality score that does not meet the threshold signal quality score.

37. The one or more non-transitory computer-readable devices of embodiment 27, wherein performing statistical analysis on the values for the first feature of the plurality of beats comprises fitting a distribution of the values for the first feature of at least some of the plurality of beats to a probability distribution function.

38. The one or more non-transitory computer-readable devices of embodiment 37, wherein the probability distribution function is a normal probability distribution function, a gamma probability distribution function, or a Gaussian probability distribution function.

39. The one or more non-transitory computer-readable devices of embodiment 27, wherein generating the indication of the level of the analyte within the human comprises comparing the values for the first feature of at least a subset of the plurality of beats to a pre-defined template that maps values for the first feature to levels of the analyte within a human or that maps values derived at least in part from the first feature to levels of the analyte within a human.

40. The one or more non-transitory computer-readable devices of embodiment 39, wherein the pre-defined template is generated based on assessments of the level of the analyte within a population of humans other than the human from which the data indicating the electrocardiogram results was obtained.

41. The one or more non-transitory computer-readable devices of embodiment 39, wherein the pre-defined template is generated based on assessments of the level of the analyte within the human from which the data indicating the electrocardiogram results was obtained.

42. The one or more non-transitory computer-readable devices of embodiment 27, wherein generating the indication of the level of the analyte within the human comprises comparing the result of the statistical analysis performed on the values for the first feature of the plurality of beats to a pre-defined template that maps statistical results for historical values for the first feature to levels of the analyte within a human.

43. The one or more non-transitory computer-readable devices of embodiment 42, wherein the pre-defined template is generated based on assessments of the level of the analyte within a population of humans other than the human from which the data indicating the electrocardiogram results was obtained.

44. The one or more non-transitory computer-readable devices of embodiment 42, wherein the pre-defined template is generated based on assessments of the level of the analyte within the human from which the data indicating the electrocardiogram results was obtained.

45. The one or more non-transitory computer-readable devices of embodiment 27, wherein the method is performed at least in part on a mobile computing device of the human.

46. The one or more non-transitory computer-readable devices of embodiment 45, wherein providing the indication of the level of the analyte within the human comprises presenting the indication of the level of the analyte on an electronic display of the mobile computing device.

47. The one or more non-transitory computer-readable devices of embodiment 27, wherein identifying the plurality of beats comprises identifying QRS complexes that occur in at least a subset of the plurality of beats, wherein identifying the QRS complexes that occur in the at least the subset of the plurality of beats comprises identifying features that occur in the electrocardiogram results at a frequency indicated by the human's heart rate or by an estimate of the R-R interval in the electrocardiogram results.

48. The one or more non-transitory computer-readable devices of embodiment 27, wherein the operations further comprise generating an animation that shows changes in the electrocardiogram results and changes in the first feature over a period of time.

49. The one or more non-transitory computer-readable devices of embodiment 27, wherein determining the values for the first feature of the plurality of beats comprises determining initial values for the first feature of the plurality of beats and filtering the initial values.

50. The one or more non-transitory computer-readable devices of embodiment 49, wherein filtering the initial values for the first feature comprises applying a Kalman filter to the initial values.

51. The one or more non-transitory computer-readable devices of embodiment 27, wherein the operations further comprise modifying the data indicating the electrocardiogram results by demodulating a QRS envelope of the electrocardiogram results.

52. One or more non-transitory computer-readable devices having instructions stored thereon that, when executed by one or more processors, cause performance of operations comprising:

generating electrocardiogram results from the human; and

comparing the results to a signal template to obtain an indication of the level of the analyte in the human,

wherein the signal template was generated based on assessments of the level of the analyte within a population of humans other than the human from which the

electrocardiogram results were generated.

53. A computing system comprising:

one or more computers; and

one or more computer-readable media having instructions stored thereon that, when executed by the one or more computers, cause performance of operations comprising:

obtaining data indicating electrocardiogram results from the human;

identifying a plurality of beats represented in the electrocardiogram results; determining, for each beat in the plurality of beats represented in the electrocardiogram results, a value for a first feature of the beat;

performing statistical analysis on the values for the first feature of the plurality of beats;

generating an indication of the level of the analyte within the human based on a result of the statistical analysis performed on the values for the first feature of the plurality of beats; and

providing the indication of the level of the analyte within the human.

54. A computing system comprising:

one or more computers; and

one or more computer-readable media having instructions stored thereon that, when executed by the one or more computers, cause performance of operations comprising:

generating electrocardiogram results from the human; and comparing the results to a signal template to obtain an indication of the level of the analyte in the human,

wherein the signal template was generated based on assessments of the level of the analyte within a population of humans other than the human from which the electrocardiogram results were generated.

**Claims**

1. An analyte level prediction apparatus comprising:

a memory; and

a processor, operatively coupled to the memory, the processing device to:

obtain electrocardiogram (ECG) data of a subject;
enter the ECG data into a machine learning model;
predict, from the machine learning model, the level of the analyte of the subject; and
provide the predicted level of the analyte of the subject.

2. The analyte level prediction apparatus of claim 1, wherein the level is a classification into a category selected from the group consisting of low, normal and high.

3. The analyte level prediction apparatus of claim 1, wherein the machine learning model comprises one or more of: a feedforward neural network, a deep convolutional neural network, a support vector machine, a Gaussian mixture model, a hidden Markov model, Bayesian decision rules, logistic regression, nearest neighbor model, and decision trees.

4. The analyte level prediction apparatus of claim 1, wherein to enter the ECG data into the machine learning model the processor is further to:

identify a plurality of beats in the ECG data; and
enter the plurality of beats into the machine learning model, wherein the machine learning model outputs a prediction of the level of the analyte.

5. The analyte level prediction apparatus of claim 1, wherein the processor is further to:

average the plurality of beats to from a representative beat; and
enter the representative beat into the machine learning model.

6. The analyte prediction apparatus of claim 1, wherein the ECG data is from only a single lead.

7. The analyte level prediction apparatus of claim 1, wherein the ECG data is from a portable ECG device that measures less than 12 leads.

8. The analyte level prediction apparatus of claim 1, wherein the analyte is potassium.

9. The analyte level prediction apparatus of claim 1, wherein the analyte is selected from a group consisting of: potassium, magnesium, phosphorous, calcium, bicarbonate, hydrogen ion, and glucose.

**10.** The analyte level prediction apparatus of claim 1, wherein the analyte level is a predicted serum analyte concentration.

**11.** The analyte level prediction apparatus of claim 1, wherein the machine learning model is trained on training ECG data of a population other than the subject from which the ECG data was obtained.

**12.** The analyte level prediction apparatus of claim 1, wherein the machine learning model is trained on training ECG data of the subject from which the ECG data was obtained.

**13.** An analyte level prediction apparatus comprising:

a memory; and
a processor, operatively coupled to the memory, the processing device to:

obtain electrocardiogram (ECG) data of a subject;
processing the ECG data to generate one or more features of the ECG data;
enter the one or more features into a statistical model;
predict, from the statistical model, the level of the analyte of the subject; and
provide the level of the analyte of the subject.

**14.** The analyte level prediction apparatus of claim 13 wherein the processor is further to:

identify a plurality of beats in the ECG data; and
determine, for each beat in the plurality of beats, a value for a first feature of each beat, wherein to predict, from the statistical model, the level of analyte, the processor is further to enter the value for the first feature of each beat into the statistical model.

**15.** The analyte level prediction apparatus of claim 14, wherein to determine the value for the first feature of each beat the processor is further to calculate, for each beat in the plurality of beats, a slope of at least a portion of a T-wave in each beat between the peak of the T-wave and the end of the T- wave.

FIG. 1

FIG. 2

FIG. 3

FIG. 4

EP 4 523 616 A2

FIG. 5A

EP 4 523 616 A2

Time: 87 min, Heart Rate : 59 bpm

502

Potassium Value

504

Normalized Feature
(-Trs/sqrt(Tamp))

506

-T Right Slope

508

Heart Rate

510

Peak

Off

FIG. 5B

EP 4 523 616 A2

Time: 240min, Heart Rate : 60bpm

Potassium Value

Normalized Feature (-Trs/sqrt(Tamp))

-T Right Slope

Heart Rate

FIG. 5C

EP 4 523 616 A2

FIG. 6B

FIG. 6A

FIG. 7

FIG. 8

## Signal averaged T wave

FIG. 9A

## T wave Pdf

FIG. 9B

## Sample Autocorrelation Function

FIG. 9C

FIG. 10

Estimation of the T amp feature PDF

FIG. 11

## Aligned ECG Beats as an Image

FIG. 12A

## Aligned ECG Beats

FIG. 12B

FIG. 13A

FIG. 13B

FIG. 14A

EP 4 523 616 A2

FIG. 14B

EP 4 523 616 A2

FIG. 15

Time: 243min, Heart Rate : 61bpm

Potassium Value

Normalized Feature
(-Trs/sqrt(Tamp))

-T Right Slope

Heart Rate

FIG. 16

EP 4 523 616 A2

K Values - Estimated
versus Lab

K Values - Estimated
versus Lab

K Values - Estimated
versus Lab

EP 4 523 616 A2

FIG. 17A

FIG. 17B

FIG. 17C

FIG. 18A

FIG. 18B

FIG. 18C

FIG. 19B

FIG. 19A

FIG. 20A       FIG. 20B       FIG. 20C

FIG. 21

2200

| | | | | | | | | | | | |
|---|---|---|---|---|---|---|---|---|---|---|---|
| HR.a | A fib | 5% | 7% | 15% | 40% | 10% | 8% | 20% | 30% | ... | | |
| | HR | 100 | 120 | 140 | 130 | 100 | 110 | 155 | 155 | ... | 90 | ... |
| Dialysis | Day | Yes | | | | | | | | | No | |
| | Time | No | | Yes | | | | No | | | No | |
| Blood Chem | mg | | | | | | | | | | ... | |
| | Na | | | ... | ↓ | ... | ↓ | ... | | | ... | |
| | Ca | | | ... | → | ... | | ... | | | ... | |
| | K+ | 7.0 | | 5.5 | ↓ | 4.8 | ↓ | 3 | | | 6 | |
| ECG Features (Per Vector) | TRS | | | | | | | | | | | |
| | ATc | | | | | | | | | | | |
| | Tamp | | | | | | | | | | | |
| | QRS.W | | | | | | | | | | | |
| | QRS.R | | | | | | | | | | | |
| | QRS.R | | | | | | | | | | | |
| Accelerometer | Est. Pos | | | | | | | | | | | |
| | B.Pushes | | | | | | | | | | | |
| | Movement | | | | | | | | | | | |
| Group | Feating | Day I | | | | | | | | | Day II | |
| Time | Hour | 0 | | 9 | | 12 | | 9 | | 0 | ... | |

FIG. 22

EP 4 523 616 A2

FIG. 23

EP 4 523 616 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62258956
- US 62401044

- WO 2015048514 A

**Non-patent literature cited in the description**

- **EINHORN LM** ; **ZHAN M** ; **WALKER LD** ; **MOEN MF** ; **SELIGER SL** ; **WEIR MR** ; **FINK JC**. The frequency of hyperkalemia and its significance in chronic kidney disease.. *Archives of Internal Medicine*, 2009, vol. 169, 1156-1162
- **GOYAL A** ; **SPERTUS JA** ; **GOSCH K**. Serum potassium levels and mortality in acute myocardial infarction. *JAMA*, 2012, vol. 307, 157-164
- **GENNARI**. *F. J. Hypokalemia. New England Journal of Medicine*, 1998, vol. 339, 451-458
- **WEINER, I. D** ; **WINGO, C. S**. Hyperkalemia: a potential silent killer. *JOURNAL-AMERICAN SOCIETY OF NEPHROLOGY*, 1998, vol. 9, 1535-1543
- **KOVESDY, C. P**. Management of hyperkalaemia in chronic kidney disease. *Nat Rev Nephrol*, 2014, vol. 10, 653-662
- **JAIN N** ; **KOTLA S** ; **LITTLE BB** ; **WEIDEMAN RA** ; **BRILAKIS ES** ; **REILLY RF** ; **BANERJEE S**. Predictors ofhyperkalemia and death in patients with cardiac and renal disease.. *The American journal of cardiology*, 2012, vol. 109, 1510-1513
- **JUURLINK DN** ; **MAMDANI MM** ; **LEE DS** ; **KOPPA** ; **AUSTIN PC** ; **LAUPACIS A** ; **REDELMEIER DA**. Rates of hyperkalemia after publication of the Randomized Aldactone Evaluation Study.. *New England Journal of Medicine*, 2004, vol. 351, 543-551
- **WILD, S** ; **ROGLIC, G** ; **GREEN, A** ; **SICREE, R.** ; **KING, H**. Global prevalence of diabetes estimates for the year 2000 and projections for 2030. *Diabetes care*, 2004, vol. 27, 1047-1053
- **ROGER VL** ; **GO AS** ; **LLOYD-JONES DM** ; **BENJAMIN EJ** ; **BERRY JD** ; **BORDEN WB** ; **BRAVATA DM** ; **DAI S** ; **FORD ES** ; **FOX CS**. American Heart Association Statistics Committee and Stroke Statistics Subcommittee. Heart Disease and Stroke Statistics-2012 Update: A Report From the American Heart Association. *Circulation*, 2012, vol. 125, e2-e220

- **GUMZ, M. L.** ; **RABINOWITZ, L.** ; **WINGO, C. S**. An Integrated View of Potassium Homeostasis.. *New England Journal of Medicine*, 2015, vol. 373, 60-72
- **J. R. A**. New Era for the Treatment of Hyperkalemia?. *New England Journal of Medicine*, 2015, vol. 372, 275-277
- **ASH, S. R.** ; **SINGH, B** ; **LAVIN, P. T.** ; **STAVROS, F** ; **RASMUSSEN, H. S**. A phase 2 study on the treatment of hyperkalemia in patients with chronic kidney disease suggests that the selective potassium trap, ZS-9, is safe and efficient. *Kidney Int,*, 2015
- **PACKHAM DK** ; **RASMUSSEN HS** ; **LAVIN PT** ; **EL-SHAHAWY MA** ; **ROGER SD** ; **BLOCK G** ; **QUNIBI W** ; **PERGOLA P** ; **SINGH B**. Sodium Zirconium Cyclosilicate in Hyperkalemia.. *New England Journal of Medicine*, 2015, vol. 372, 222-231
- **BAKRIS GL** ; **PITT B** ; **WEIR MR** ; **FREEMAN MW** ; **MAYO MR** ; **GARZA D** ; **STASIV Y** ; **ZAWADZKI R** ; **BERMAN L** ; **BUSHINSKY DA**. AMETHYST-DN Investigators. Effect of patiromer on serum potassium level in patients with hyperkalemia and diabetic kidney disease: The amethyst-dn randomized clinical trial.. *JAMA*, 2015, vol. 314, 151-161
- **ROSCIONI, S. S.** ; **HEERSPINK, H. J. L**. Clinical trials: New nonabsorbable potassium-exchange resins in hyperkalaemia. *Nat Rev Nephrol*, 2015, vol. 11, 205-206
- **DILLON JJ** ; **DESIMONE CV** ; **SAPIR Y** ; **SOMERS VK** ; **DUGAN JL** ; **BRUCE CJ** ; **ACKERMAN MJ** ; **ASIRVATHAM SJ** ; **STRIEMER BL** ; **BUKARTYK J**. Noninvasive potassium determination using a mathematically processed ECG: Proof of concept for a novel "blood-less, blood test. *Journal of electrocardiology*, 2015, vol. 48, 12-18
- **ZIDELMAL, Z.** ; **AMIROU, A** ; **ADNANE, M** ; **BELOUCHRANI, A**. QRS detection based on wavelet coefficients.. *Comput. Methods Prog. Biomed.*, 2012, vol. 107, 490-496

- **SEENA, V.** ; **YOMAS, J.** *Devices, Circuits and Systems (ICDCS), 2014 2nd International Conference on*, 1-6
- **HAMMING, R**. Numerical methods for scientists and engineers. *Courier Corporation*, 2012
- **ZHANG, Q** ; **MANRIQUEZ, A. I.** ; **MEDIGUE, C** ; **PAPELIER, Y.** ; **SORINE, M**. An algorithm for robust and efficient location of T-wave ends in electrocardiograms.. *Biomedical Engineering, IEEE Transactions on*, 2006, vol. 53, 2544-2552
- **HELFENBEIN ED** ; **ACKERMAN MJ** ; **RAUTAHARJU PM** ; **ZHOU SH** ; **GREGG RE** ; **LINDAUER JM** ; **MILLER D** ; **WANG JJ** ; **KRESGE SS** ; **BABAEIZADEH S**. An algorithm for QT interval monitoring in neonatal intensive care units.. *Journal of electrocardiology*, 2007, vol. 40, S103-S110
- **KALMAN, R. E.** A new approach to linear filtering and prediction problems. *Journal of Fluids Engineering*, 1960, vol. 82, 35-45
- **GENNARI**. F. J. Hypokalemia. *New England Journal of Medicine*, 1998, vol. 339, 451-458
- **WEINER, I. D.** ; **WINGO, C. S**. Hyperkalemia: a potential silent killer.. *JOURNAL-AMERICAN SOCIETY OF NEPHROLOGY*, 1998, vol. 9, 1535-1543
- **INGELFINGER, J. R**. A New Era for the Treatment of Hyperkalemia?. *New England Journal of Medicine*, 2015, vol. 372, 275-277
- **ASH, S. R** ; **SINGH, B.** ; **LAVIN, P. T** ; **STAVROS, F.** ; **RASMUSSEN, H. S**. A phase 2 study on the treatment of hyperkalemia in patients with chronic kidney disease suggests that the selective potassium trap, ZS-9, is safe and efficient.. *Kidney Int,*, 2015
- **PACKHAM DK** ; **RASMUSSEN HS** ; **LAVIN PT** ; **EL-SHAHAWY MA** ; **ROGER SD** ; **BLOCK G** ; **QUNIBI W** ; **PERGOLA P** ; **SINGH B**. Sodium Zirconium Cyclosilicate in Hyperkalemia. *New England Journal of Medicine*, 2015, vol. 372, 222-231
- **KOSIBOROD M** ; **RASMUSSEN HS** ; **LAVIN P** ; **QUNIBI WY** ; **SPINOWITZ B** ; **PACKHAM D** ; **ROGER SD** ; **YANG A** ; **LERMA E** ; **SINGH B.** Effect of sodium zirconium cyclosilicate on potassium lowering for 28 days among outpatients with hyperkalemia: The harmonize randomized clinical trial.. *JAMA*, 2014, vol. 312, 2223-2233
- **DILLON JJ** ; **DESIMONE CV** ; **SAPIR Y** ; **SOMERS VK** ; **DUGAN JL** ; **BRUCE CJ** ; **ACKERMAN MJ** ; **ASIRVATHAM SJ** ; **STRIEMER BL** ; **BUKARTYK J**. Noninvasive potassium determination using a mathematically processed ECG: Proof of concept for a novel ''blood-less, blood test''.. *Journal of electrocardiology*, 2015, vol. 48, 12-18
- **YAN, G.-X.** ; **ANTZELEVITCH, C**. Cellular basis for the normal T wave and the electrocardiographic manifestations of the long-QT syndrome.. *Circulation*, 1998, vol. 98, 1928-1936
- **CHOE, S**. Potassium channel structures. *Nature Reviews Neuroscience*, 2002, vol. 3, 115-121
- **CORSI C** ; **DEBIE J** ; **NAPOLITANO C** ; **PRIORI S** ; **MORTARA D** ; **SEVERI S**. Validation of a novel method for non-invasive blood potassium quantification from the ECG.. *Computing in Cardiology (CinC)*, 2012, 105-108
- **KANIA M** ; **RIX H** ; **FERENIEC M** ; **ZAVALA-FERNANDEZ H** ; **JANUSEK D** ; **MROCZKA T,** ; **STIX G** ; **MANIEWSKI R**. The effect of precordial lead displacement on ECG morphology.. *Medical & biological engineering & computing*, 2014, vol. 52, 109-119
- **NGUYÊN UC** ; **POTSE M** ; **REGOLI F** ; **CAPUTO ML** ; **CONTE G** ; **MURZILLI R** ; **MUZZARELLI S** ; **MOCCETTI T** ; **CAIANI EG** ; **PRINZEN FW**. An in-silico analysis of the effect of heart position and orientation on the ECG morphology and vectorcardiogram parameters in patients with heart failure and intraventricular conduction defects. *Journal of Electroccardiology*, 2015